# EUROPEAN PATENT APPLICATION

(11) **EP 1 375 663 A1**
(43) Date of publication of application: **02.01.2004**
(21) Application number: 02705476.6
(22) Date of filing: 25.03.2002
(51) Int. Cl.: C12N 15/12, C07K 14/47, C07K 16/18, C12N 5/10, C12P 21/02, C12P 21/08, C12Q 1/02, A61K 39/395, A61K 45/00, A61K 48/00, A61P 9/00, A61P 9/08, A61P 9/10, A61P 25/00, G01N 33/15, G01N 33/50, G01N 33/68

(54) **NOVEL PROTEIN, DNA THEREOF AND USE OF THE SAME**

(30) Priority: 26.03.2001 JP 2001088041; 28.08.2001 JP 2001258443
(71) Applicant: Takeda Chemical Industries, Ltd., Osaka-shi, Osaka 541-0045 (JP)
(72) Inventor: HORIGUCHI, Takashi, Ibaraki 305-0035 (JP); TANIDA, Seiichi, Nagaokakyo-shi, Kyoto 617-0857 (JP)
(74) Representative: Lewin, John Harvey
(86) International application number: PCT/JP2002/002841
(87) International publication number: WO 2002/081690

(57) **Abstract**

The present invention provides a novel protein, a DNA thereof, a screening method with the use of the same, and so on. A compound or a salt thereof regulating the activity of the present protein is useful as a prophylactic and/or therapeutic agent for heart diseases and central nervous system diseases.

## Description

### TECHNICAL FIELD

The present invention relates to a novel rat-derived protein, a DNA thereof, and uses thereof, more specifically, a method of screening for a prophylactic and/or therapeutic agent for heart diseases and central nervous system diseases.

### BACKGROUND ART

MEF(myocyte enhancer factor)-2C is an isoform of MEF family consisting of transcription control factors that bind to MADS (MCM1, agamous, deficiens, serum-response factor)-box sequence, and strong expression of MEF-2C is recognized in the heart, skeletal muscle, brain, spleen, etc. (Proc. Natl. Acad. Sci. USA 90: 5282-5286, 1993; Science 276: 1404-1407, 1997). MEF-2C knockout mice are embryolethal and exhibit hypoplasia of the heart, and in these mice, the expression of genes such as atrial natriuretic peptide gene and α myosin heavy chain gene that play important roles in the function of heart is remarkably decreased (Science 276: 1404-1407, 1997). Further, MEF-2C is expressed specifically in central nerve cells whose division has been completed, and plays an important role in the survival of those cells (Science 286: 785-790; 1999). Further, MEF-2C and a nerve-specific transcription factor MASH-1 both synergistically enhance the expression of nerve-specific genes (FEBS Letters, 472: 53-56,2000).

From these findings, it is clear that MEF-2C plays an important role in the development, differentiation, or maintenance of the function of the heart and the central nerve system, and it is considered that the malfunction of MEF-2C is involved in a pathology of a heart disease or a central nerve disease. Therefore, it is believed that compounds that regulate the function of MEF-2C may be used as prophylactics and/or therapeutics for heart diseases and central nerve diseases. However, no compounds that regulate the function of MEF-2C have been reported yet.

To date, for example, cardiotonics, sympathomimetics, vasodilators, blockers and the like have been used to treat heart diseases, but they are not sufficiently effective and safe. It is desired to develop new prophylactics or therapeutics for heart diseases and the like with sufficient effectiveness and safety.

### DISCLOSURE OF THE INVENTION

As a result of intensive and extensive researches toward the solution of the above-described problem, the present inventors have cloned rat MEF-2C that is considered to control the expression of a group of genes which play important roles for maintaining the functions of the heart and the central nerve system. Further, in order to obtain novel prophylactics and/or therapeutics for heart diseases or central nerve diseases, the inventors have established methods of screening for compounds that regulate the activity of the above-mentioned gene product protein. A further study based on these findings made us achieve the invention.

The invention provides:
(1) A protein comprising the same or substantially the same amino acid sequence as the amino acid sequence shown by SEQ ID NO: 1, 2, 3, 15, 16 or 17; or a salt thereof.
(2) The protein according to (1) or a salt thereof, which comprises the same or substantially the same amino acid sequence as the amino acid sequence shown by SEQ ID NO: 1, 2 or 3.
(3) The protein according to (1) or a salt thereof, which comprises the amino acid sequence shown by SEQ ID NO: 1.
(4) The protein according to (1) or a salt thereof, which comprises the amino acid sequence shown by SEQ ID NO: 2.
(5) The protein according to (1) or a salt thereof, which comprises the amino acid sequence shown by SEQ ID NO: 3.
(6) The protein according to (1) or a salt thereof, which comprises the amino acid sequence shown by SEQ ID NO: 15.
(7) The protein according to (1) or a salt thereof, which comprises the amino acid sequence shown by SEQ ID NO: 16.
(8) The protein according to (1) or a salt thereof, which comprises the amino acid sequence shown by SEQ ID NO: 17.
(9) A partial peptide of the protein according to (1), or a salt thereof.
(10) A polynucleotide comprising a polynucleotide encoding the protein according to (1) or the partial peptide according to (9).
(11) The polynucleotide according to (10), which is DNA.
(12) The polynucleotide according to (11), which comprises the nucleotide sequence represented by SEQ ID NO: 4, 5, 6, 18, 19 or 20.
(13) A recombinant vector comprising the polynucleotide according to (10).
(14) A transformant transformed by the recombinant vector according to (13).
(15) A method of producing the protein according to (1) or a salt thereof, or the partial peptide according to (9) or a salt thereof, which comprises culturing the transformant according to (14) to produce and accumulate the protein according to (1) or the partial peptide according to (9); and collecting it.
(16) An antibody to the protein according to (1) or a salt thereof or the partial peptide according to (9) or a salt thereof.
(17) A pharmaceutical composition comprising the antibody according to (16).
(18) A diagnostic composition comprising the antibody according to (16).
(19) A method of screening for a compound or a salt thereof regulating the activity of the protein according to (1) or a salt thereof or the partial peptide according to (9) or a salt thereof, which comprises using the protein according to (1) or a salt thereof or the partial peptide according to (9) or a salt thereof.
(20) A kit for screening for a compound or a salt thereof regulating the activity of the protein according to (1) or a salt thereof or the partial peptide according to (9) or a salt thereof, which comprises the protein according to (1) or a salt thereof or the partial peptide according to (9) or a salt thereof.
(21) A compound or a salt thereof regulating the activity of the protein according to (1) or a salt thereof or the partial peptide according to (9) or a salt thereof, which can be obtained by the screening method according to (19) or the screening kit according to (20).
(22) A pharmaceutical composition comprising the compound according to (21) or a salt thereof.
(23) A method of screening for a compound or a salt thereof regulating the gene expression of the protein according to (1 ), which comprises using the polynucleotide according to (10).
(24) A method of screening for a compound or a salt thereof enhancing the gene expression of the protein comprising the same or substantially the same amino acid sequence as the amino acid sequence shown by SEQ ID NO: 1 or 15, which comprises using a polynucleotide encoding the said protein.
(25) A method of screening for a compound or a salt thereof inhibiting the gene expression of the protein comprising the same or substantially the same amino acid sequence as the amino acid sequence shown by SEQ ID NO: 2, 3, 16 or 17, which comprises using a polynucleotide encoding the said protein.
(26) A kit for screening for a compound or a salt thereof regulating the gene expression of the protein according to (1), which comprises the polynucleotide according to (10).
(27) A compound or a salt thereof regulating the gene expression of the protein according to (1), which can be obtained by the screening method according to (23) or the screening kit according to (26).
(28) A compound or a salt thereof enhancing the gene expression of the protein comprising the amino acid sequence shown by SEQ ID NO: 1 or 15, which can be obtained by the screening method according to (24).
(29) A compound or a salt thereof inhibiting the gene expression of the protein comprising the amino acid sequence shown by SEQ ID NO: 2, 3, 16 or 17, which can be obtained by the screening method according to (25).
(30) A pharmaceutical composition comprising the compound according to (27), (28) or (29) or a salt thereof.
(31 ) An antisense polynucleotide comprising a nucleotide sequence complementary to the polynucleotide according to (10) or a partial sequence thereof.
(32) A pharmaceutical composition comprising the antisense polynucleotide according to (31 ).
(33) The pharmaceutical composition according to (17), (22), (30) or (32), which is a prophylactic and/or therapeutic agent for heart diseases or central nervous system diseases.
(34) A method of preventing and/or treating heart diseases or central nervous system diseases in a mammal, which comprises administering an effective amount of the compound according to (21), (27), (28) or (29) or a salt thereof to the mammal.
(35) A use of the compound according to (21), (27), (28) or (29) or a salt thereof for producing a prophylactic and/or therapeutic agent for heart diseases or central nervous system diseases.

### BRIEF DESCRIPTION OF THE DRAWINGS

Fig. 1 shows the changes of the expressing amounts of MEF2ChW gene and MEF2ChV 1 gene after the replacement with a serum-free medium.
Fig. 2 shows the effect of MEF2ChW gene or MEF2ChV1 gene on the expression of Atrial Natriuretic Peptide gene. In this figure, the ordinate axis indicates the fluorescence intensity and the abscissa axis indicates genes (plasmids) used.
Fig. 3 shows the changes of the expressing amounts of MEF2ChW gene and MEF2ChV1 gene after addition of tunicamycin.
Fig. 4 shows the effect of MEF2ChW gene or MEF2ChV 1 gene on the expression of N-methyl-D-aspartate receptor gene. In this figure, the ordinate axis indicates the fluorescence intensity and the abscissa axis indicates genes (plasmids) used.

### BEST MODE FOR CARRYING OUT THE INVENTION

The protein of the invention comprising the same or substantially the same amino acid sequence as the amino acid sequence represented by SEQ ID NO: 1, 2, 3, 15, 16 or 17 (sometimes referred to the protein of the invention or the protein used in the invention) may be derived from any cells of a human or non-human warm-blooded animals (e.g. guinea pig, rat, mouse, chicken, rabbit, swine, sheep, bovine, monkey) (e.g. hepatocytes, splenocytes, nerve cells, glial cells, pancreatic cells, bone marrow cells, mesangial cells, Langerhans' cells, epidermic cells, epithelial cells, beaker cell, endothelial cells, smooth muscle cells, fibroblasts, fibrocytes, myocytes, fat cells, immune cells (e.g. macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells); or any tissues where such cells are present, such as brain or any brain regions (e.g. olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g. large intestine and small intestine), blood vessel, heart, thymus, spleen, submandibular gland, peripheral blood, prostate, testis, ovary, placenta, uterus, bone, joint, skeletal muscle, etc; the protein may also be a synthetic protein.

The amino acid sequence which is substantially the same as represented by SEQ ID NO: 1, 2, 3, 15, 16 or 17 includes an amino acid sequence having at least about 70% homology, preferably at least about 80% homology, and preferably at least about 90% homology to the amino acid sequence represented by SEQ ID NO: 1, 2, 3, 15, 16 or 17, respectively.

Preferably, the protein comprising substantially the same amino acid sequence as represented by SEQ ID NO: 1, 2, 3, 15, 16 or 17 includes the protein comprising substantially the same amino acid sequence as represented by SEQ ID NO: 1, 2, 3, 15, 16 or 17 and having an activity substantially the same in property as that of the protein comprising the amino acid sequence represented by SEQ ID NO: 1, 2, 3, 15, 16 or 17. But, the protein of the invention excludes the protein having the amino acid sequence represented by SEQ ID NO: 21 (human MEF-2C; Proc. Natl. Acad. Sci. U.S.A. 90, No. 4, 1546-1550, 1993) and the protein having the amino acid sequence represented by SEQ ID NO: 22 (mouse MEF-2C; Proc. Natl. Acad. Sci. U.S.A. 90, 5282-5286, 1993), and also the protein having the amino acid sequence represented by SEQ ID NO: 23. (GenBank Accession No. AK009139; the splicing variant of mouse MEF-2C).

More preferably, the amino acid sequence which is substantially the same as represented by SEQ ID NO: 1 includes an amino acid sequence having at least about 99% homology, preferably at least about 99.5% homology to the amino acid sequence represented by SEQ ID NO: 1.

Preferably, the protein comprising substantially the same amino acid sequence as represented by SEQ ID NO: 1 includes the protein comprising substantially the same amino acid sequence as represented by SEQ ID NO: 1 and having an activity substantially the same in property as that of the protein comprising the amino acid sequence represented by SEQ ID NO: 1.

The amino acid sequence which is substantially the same as represented by SEQ ID NO: 2 includes an amino acid sequence having at least about 93% homology, preferably at least about 96% homology, more preferably at least about 99% homology to the amino acid sequence represented by SEQ ID NO: 2.

Preferably, the protein comprising substantially the same amino acid sequence as represented by SEQ ID NO: 2 includes the protein comprising substantially the same amino acid sequence as represented by SEQ ID NO: 2 and having an activity substantially the same in property as that of the protein comprising the amino acid sequence represented by SEQ ID NO: 2.

The amino acid sequence which is substantially the same as represented by SEQ ID NO: 3 includes an amino acid sequence having at least about 90% homology, preferably at least about 95% homology, more preferably at least about 97% homology to the amino acid sequence represented by SEQ ID NO: 3.

Preferably, the protein comprising substantially the same amino acid sequence as represented by SEQ ID NO: 3 includes the protein comprising substantially the same amino acid sequence as represented by SEQ ID NO: 3 and having an activity substantially the same in property as that of the protein comprising the amino acid sequence represented by SEQ ID NO: 3.

The amino acid sequence which is substantially the same as represented by SEQ ID NO: 15 includes an amino acid sequence having at least about 99.5% homology, preferably at least about 99.9% homology to the amino acid sequence represented by SEQ ID NO: 15.

Preferably, the protein comprising substantially the same amino acid sequence as represented by SEQ ID NO: 15 includes the protein comprising substantially the same amino acid sequence as represented by SEQ ID NO: 15 and having an activity substantially the same in property as that of the protein comprising the amino acid sequence represented by SEQ ID NO: 15.

The amino acid sequence which is substantially the same as represented by SEQ ID NO: 16 includes an amino acid sequence having at least about 93% homology, preferably at least about 97% homology to the amino acid sequence represented by SEQ ID NO: 16.

Preferably, the protein comprising substantially the same amino acid sequence as represented by SEQ ID NO: 16 includes the protein comprising substantially the same amino acid sequence as represented by SEQ ID NO: 16 and having an activity substantially the same in property as that of the protein comprising the amino acid sequence represented by SEQ ID NO: 16.

The amino acid sequence which is substantially the same as represented by SEQ ID NO: 17 includes an amino acid sequence having at least about 85% homology, preferably at least about 90% homology, more preferably at least about 95% homology to the amino acid sequence represented by SEQ ID NO: 17.

Preferably, the protein comprising substantially the same amino acid sequence as represented by SEQ ID NO: 17 includes the protein comprising substantially the same amino acid sequence as represented by SEQ ID NO: 17 and having an activity substantially the same in property as that of the protein comprising the amino acid sequence represented by SEQ ID NO: 17.

The above "activity substantially the same in property" is exemplified by the activity of enhancing the cardiac function. The term "substantially the same in property" means that the activity is identical in property (e.g. physiologically or pharmacologically). Thus, it is preferable that the activity of enhancing the cardiac function shows the same level (e.g. about 0.01 to 100 folds, preferably about 0.1 to 10 folds, more preferably about 0.5 to 2 folds), however, any quantitative factors such as a level of this activity and a molecular weight of the proteins may be different.

The activity of enhancing the cardiac function can be measured by an echocardiographic device (Cell, Vol. 97: 189-198, 1999) or cardiac function measurement using a cardiac catheter (Circulation Research 69: 370-377, 1991). Moreover, the said activity can be determined by, for example, measurement of activation of renin-angiotensin system (RAS) such as angiotensin I-converting enzyme (ACE) using a commercial kit (e.g. made by Peninsula Corp., Phoenix Corp., etc) or measurement of an increase in blood catecholamine (full automatic catecholamine analyzer, Toso) as an index.

The protein of the invention also includes so-called muteins, for example:
(1) a protein comprising an amino acid sequence represented by SEQ ID NO: 1, in which one or more (preferably 1 to 30, more preferably 1 to 10, and even more preferably 1 to 5) amino acids are deleted; an amino acid sequence represented by SEQ ID NO: 1, to which one or more (preferably 1 to 30, more preferably 1 to 10, and even more preferably 1 to 5) amino acids are added; an amino acid sequence represented by SEQ ID NO: 1, into which one or more (preferably 1 to 5) amino acids are inserted; an amino acid sequence represented by SEQ ID NO: 1, in which one or more (preferably 1 to 5) amino acids are substituted by other amino acids; or an amino acid sequence represented by SEQ ID NO: 1 having a combination of the above modifications;
(2) a protein comprising an amino acid sequence represented by SEQ ID NO: 2, in which one or more (preferably 1 to 30, more preferably 1 to 10, and even more preferably 1 to 5) amino acids are deleted; an amino acid sequence represented by SEQ ID NO: 2, to which one or more (preferably 1 to 30, more preferably 1 to 10, and even more preferably 1 to 5) amino acids are added; an amino acid sequence represented by SEQ ID NO: 2, into which one or more (preferably 1 to 10, more preferably 1 to 5) amino acids are inserted; an amino acid sequence represented by SEQ ID NO: 2, in which one or more (preferably 1 to 10, more preferably I to 5) amino acids are substituted by other amino acids; or an amino acid sequence represented by SEQ ID NO: 2 having a combination of the above modifications;
(3) a protein comprising an amino acid sequence represented by SEQ ID NO: 3, in which one or more (preferably 1 to 30, more preferably 1 to 10, and even more preferably 1 to 5) amino acids are deleted; an amino acid sequence represented by SEQ ID NO: 3, to which one or more (preferably 1 to 30, more preferably 1 to 10, and even more preferably 1 to 5) amino acids are added; an amino acid sequence represented by SEQ ID NO: 3, into which one or more (preferably 1 to 30, more preferably 1 to 10, and even more preferably 1 to 5) amino acids are inserted; an amino acid sequence represented by SEQ ID NO: 3, in which one or more (preferably 1 to 30, more preferably 1 to 10, and even more preferably 1 to 5) amino acids are substituted by other amino acids; or an amino acid sequence represented by SEQ ID NO: 3 having a combination of the above modifications;
(4) a protein comprising an amino acid sequence represented by SEQ ID NO: 15, in which one or more (preferably 1 to 30, more preferably 1 to 10, and even more preferably 1 to 5) amino acids are deleted; an amino acid sequence represented by SEQ ID NO: 15, to which one or more (preferably 1 to 30, more preferably 1 to 10, and even more preferably 1 to 5) amino acids are added; an amino acid sequence represented by SEQ ID NO: 15, into which one or more (preferably 1 to 30, more preferably 1 to 10, and even more preferably 1 to 5) amino acids are inserted; an amino acid sequence represented by SEQ ID NO: 15, in which one or more (preferably 1 to 30, more preferably 1 to 10, and even more preferably 1 to 5) amino acids are substituted by other amino acids; or an amino acid sequence represented by SEQ ID NO: 15 having a combination of the above modifications;
(5) a protein comprising an amino acid sequence represented by SEQ ID NO: 16, in which one or more (preferably 1 to 30, more preferably 1 to 10, and even more preferably 1 to 5) amino acids are deleted; an amino acid sequence represented by SEQ ID NO: 16, to which one or more (preferably 1 to 30, more preferably 1 to 10, and even more preferably 1 to 5) amino acids are added; an amino acid sequence represented by SEQ ID NO: 16, into which one or more (preferably 1 to 30, more preferably 1 to 10, and even more preferably 1 to 5) amino acids are inserted; an amino acid sequence represented by SEQ ID NO: 16, in which one or more (preferably 1 to 30, more preferably 1 to 10, and even more preferably 1 to 5) amino acids are substituted by other amino acids; or an amino acid sequence represented by SEQ ID NO: 16 having a combination of the above modifications;
(6) a protein comprising an amino acid sequence represented by SEQ ID NO: 17, in which one or more (preferably 1 to 30, more preferably 1 to 10, and even more preferably 1 to 5) amino acids are deleted; an amino acid sequence represented by SEQ ID NO: 17, to which one or more (preferably 1 to 30, more preferably 1 to 10, and even more preferably 1 to 5) amino acids are added; an amino acid sequence represented by SEQ ID NO: 17, into which one or more (preferably 1 to 30, more preferably 1 to 10, and even more preferably 1 to 5) amino acids are inserted; an amino acid sequence represented by SEQ ID NO: 17, in which one or more (preferably 1 to 30, more preferably 1 to 10, and even more preferably 1 to 5) amino acids are substituted by other amino acids; or an amino acid sequence represented by SEQ ID NO: 17 having a combination of the above modifications.

In the present specification, proteins are shown in accordance with the conventional peptide notation with the N-terminal (amino terminal) on the left side and the C-terminal (carboxyl terminal) on the right side. The protein of the invention may have the C-terminal in a form of a carboxyl group (-COOH), a carboxylate (-COO⁻), an amide (-CONH₂) or an ester (-COOR).

Examples of the ester group shown by R include a C₁₋₆ alkyl group such as methyl, ethyl, n-propyl, isopropyl, n-butyl; a C₃₋₈ cycloalkyl group such as cyclopentyl, cyclohexyl; a C₆₋₁₂ aryl group such as phenyl, α-naphthyl; a C₇₋₁₄ aralkyl such as a phenyl-C₁₋₂ alkyl group, e.g. benzyl, phenethyl, and an α-naphthyl-C₁₋₂ alkyl group, e.g. α-naphthylmethyl; pivaloyloxymethyl, and the like.

When the protein of the invention contains a carboxyl group (or a carboxylate) at a position other than the C-terminal, it may be amidated or esterified. Such an amide or ester is also. included within the protein of the invention. This ester group may be the same as the above-mentioned C-terminal ester group.

Furthermore, examples of the protein of the invention include variants of the above proteins, wherein the amino group of the N-terminal amino acid (e.g. methionine residue) is protected with a protecting group (e.g. C₁₋₆ acyl such as C₁₋₆ alkanoyl, e.g. formyl, acetyl); those wherein the N-terminal glutamyl group newly formed after cleavage in vivo is pyroglutaminated; those wherein a substituent (e.g. -OH, -SH, amino group, imidazole group, indole group, guanidino group) on the side chain of an amino acid in the molecule is protected with a suitable protecting group (e.g. C₁₋₆ acyl such as C₁₋₆ alkanoyl, e.g. formyl, acetyl); or conjugated proteins such as glycoproteins having sugar chains.

Examples of the protein of the invention include the protein comprising the amino acid sequence represented by SEQ ID NO: 1, the protein comprising the amino acid sequence represented by SEQ ID NO: 2, the protein comprising the amino acid sequence represented by SEQ ID NO: 3, the protein comprising the amino acid sequence represented by SEQ ID NO: 15, the protein comprising the amino acid sequence represented by SEQ ID NO: 16, and the protein comprising the amino acid sequence represented by SEQ ID NO: 17.

The partial peptide of the protein of the invention (sometimes referred to as the partial peptide of the invention) may be a partial peptide of any one of the proteins of the invention described above, preferably one having an activity similar to that of the proteins of the invention described above. Further, the partial peptide of the invention includes an amide or an ester thereof.

For example, used are peptides having at least 20, preferably at least 50, more preferably at least 70, much more preferably at least 100 and most preferably at least 150 amino acid residues in the constitutional amino acid sequence of the protein of the invention.

In the partial peptide of the invention, one or more (preferably 1 to 20, more preferably 1 to 10, even more preferably 1 to 5) amino acids in the amino acid sequence may be deleted; one or more (preferably 1 to 20, more preferably 1 to 10, even more preferably 1 to 5) amino acids may be added to the amino acid sequence; one or more (preferably 1 to 20, more preferably 1 to 10, even more preferably 1 to 5) amino acids may be inserted into the amino acid sequence; or one or more (preferably 1 to 10, more preferably several, even more preferably 1 to 5) amino acids in the amino acid sequence may be substituted by other amino acids.

In the partial peptide of the invention, the C-terminal usually has a form of carboxyl group (-COOH) or carboxylate (-COO⁻). Like the protein of the invention as described above, an amide form (-CONH₂) or an ester form (-COOR) is also possible (R is defined as above). In addition, when the partial peptide of the invention contains a carboxyl group (or a carboxylate) at a position other than the C-terminal, it may be amidated or esterified. Such an amide or ester is also included within the partial peptide of the invention. This ester group may be the same as the above-mentioned C-terminal ester group.

Furthermore, like the protein of the invention as described above, the partial peptide of the invention includes variants of the above peptides, in which the amino group of the N-terminal amino acid (e.g. methionine residue) is protected by a protecting group; those in which the N-terminal glutamine residue, newly formed by cleavage in vivo, is pyroglutaminated; those in which a substituent on the side chain of an amino acid in the molecule is protected by a suitable protecting group; or a conjugated peptide such as a so-called glycopeptide bound to sugar chains.

The partial peptide of the invention can be used as an antigen for producing an antibody.

Examples of the partial peptide of the invention include the peptide having the partial amino acid sequence from 360th to 391st positions from the N-terminal in the amino acid sequence represented by SEQ ID NO: 1; the peptide having the partial amino acid sequence from 87th to 134th positions, from 271 st to 278th positions, or from 368th to 399th positions from the N-terminal in the amino acid sequence represented by SEQ ID NO: 15.

The salt of the protein or the partial peptide of the invention may be a salt with physiologically acceptable acids (e.g. inorganic acids, organic acids) or bases (e.g. alkaline metals), and physiologically acceptable acid addition salts are particularly preferred. Examples of such salts are salts with inorganic acids (e.g. hydrochloric acid, phosphoric acid, hydrobromic acid, sulfuric acid), and salts with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The protein of the invention, the partial peptide or the salt thereof may be produced by a known method for protein purification from human or non-human warm-blooded animal tissues or cells described above, or may be produced by culturing a transformant containing a DNA encoding the protein, or may also be produced by the peptide synthesis method described below.

To produce the protein of the invention, the partial peptide or the salt thereof from human or non-human warm-blooded animal tissues or cells, after these tissues or cells are homogenized and extracted with an acid, the protein is isolated or purified from the obtained extract by a combination of chromatography techniques such as reversed phase chromatography, ion exchange chromatography, and the like.

To synthesize the protein of the invention, the partial peptide thereof, the salt thereof, or the amide thereof, commercially available resins for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-arninoethyl) phenoxy resin. Using these resins, amino acids in which α-amino groups and functional groups on the side chains are appropriately protected are condensed on the resin in the order of the sequence of the objective protein according to various condensation methods publicly known. At the end of the reaction, the protein or the partial peptide is excised from the resin and at the same time, the protecting groups are removed. Then, intramolecular disulfide bond-forming reaction is performed in a highly diluted solution to obtain the objective protein or partial peptide, or an amide thereof.

For condensation of the protected amino acids described above, a variety of activation reagents for protein synthesis may be used, but carbodiimides are particularly preferably employed. Examples of such carbodiimides include DCC, N,N'-diisopropylcarbodiimide, N-ethyl-N'-(3-dimethylaminopropyl)carbodiimide. For activation by these reagents, the protected amino acids in combination with a racemization inhibitor (e.g. HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents used to activate the protected amino acids or condense with the resin may be chosen from solvents that are known to be usable for protein condensation reactions. For example, used are acid amides such as N,N-dimethylformamide, N,N-dimethylacetamide, N-methylpyrrolidone; halogenated hydrocarbons such as methylene chloride, chloroform; alcohols such as trifluoroethanol; sulfoxides such as dimethylsulfoxide; ethers such as pyridine, dioxane, tetrahydrofuran; nitriles such as acetonitrile, propionitrile; esters such as methyl acetate, ethyl acetate; and appropriate mixtures of these solvents. The reaction temperature is appropriately chosen from the range known to be applicable to protein binding reactions and is usually selected in the range of about -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of groups for protecting an amino group in the starting material include Z, Boc, t-pentyloxycarbonyl, isobomyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc.

A carboxyl group can be protected by e.g. alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl), aralkyl esterification (e.g. esterification in the form of benzyl ester, 4-nitrobenzyl ester, 4-methoxybenzyl ester, 4-chlorobenzyl ester, benzhydryl ester), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group, t-butyl group.

Examples of groups for protecting the phenolic hydroxyl group of tyrosine include Bzl, Cl₂-Bzl, 2-nitrobenzyl, Br-Z, t-butyl.

Examples of groups for protecting the imidazole moiety of histidine include Tos, 4-methoxy-2,3,6-trimethylbenzenesulfonyl, DNP, benzyloxymethyl, Bum, Boc, Trt, Fmoc.

Examples of the activated carboxyl group in the starting material include the corresponding acid anhydrides, azides, activated esters [esters with alcohols (e.g. pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. Examples of the activated amino groups in the starting material include the corresponding phosphoric amide.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of about -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups in the starting material, which should not be involved in the reaction, protecting groups, elimination of the protecting groups and activation of functional groups involved in the reaction may be appropriately selected from publicly known groups and publicly known means.

In another method for obtaining an amide of the protein or the partial peptide, for example, the α-carboxyl group of the carboxy terminal amino acid is first protected by amidation; the peptide (protein) chain is then extended to amino group for a desired length. Thereafter, the protein or the partial peptide in which only the protecting group of the N-terminal α-amino group has been eliminated from the protein or the partial peptide in which only the protecting group of the C-terminal carboxyl group has been eliminated are manufactured. The protein or the peptide is condensed in a mixture of the solvents described above. The details of the condensation reaction are the same as described above. After the protected protein or the peptide obtained by the condensation is purified, all the protecting groups are eliminated by the method described above to give the desired crude protein or peptide. This crude protein or peptide is purified by various known purification means. Lyophilization of the maj or fraction gives the desired amide of the protein or peptide.

To prepare the esterified protein or peptide, for example, the α-carboxyl group of the carboxy terminal amino acid is condensed with a desired alcohol to prepare the amino acid ester, which is then processed by procedure similar to the preparation of the amidated protein or peptide above to give the desired esterified protein or peptide.

The protein or partial peptide of the invention or a salt thereof can be produced by publicly known methods for peptide synthesis, or by cleaving the protein of the invention with an appropriate peptidase. For the peptide synthesis methods, for example, either solid phase synthesis or liquid phase synthesis may be used. Thus, a partial peptide or amino acids that can construct the partial peptide of the invention can be condensed with the remaining part of the partial peptide of the invention. When the product contains protecting groups, these protecting groups are removed to give the desired peptide. Publicly known methods for condensation and elimination of the protecting groups are described in the following 1) to 5.)
1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: Peptide Gosei-no-Kiso to Jikken (Basics and experiments of peptide synthesis), published by Maruzen Co. (1975)
4) Haruaki Yajima & Shunpei Sakakibara: Seikagaku Jikken Koza (Biochemical Experiment) 1, Tanpakushitsu no Kagaku (Chemistry of Proteins) IV, 205 (1977)
5) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the partial peptide of the invention can be purified or isolated by a combination of conventional purification methods such as solvent extraction, distillation, column chromatography, liquid chromatography and recrystallization to give the partial peptide of the invention. When the partial peptide obtained by the above methods is in a free form, it can be converted into an appropriate salt by a publicly known method; when the partial peptide is obtained in a salt form, it can be converted into a free form or a different salt form by the publicly known method.

The polynucleotide encoding the protein of the invention may be any one comprising the nucleotide sequence encoding the above-mentioned protein of the invention. Preferably, it may be a DNA. The DNA may be derived from a genome DNA, a genome DNA library, cDNAs derived from the aforementioned tissues and cells, a cDNA library derived from the aforementioned tissues and cells, or synthetic DNAs.

Vectors used for the libraries may be any one of bacteriophage, plasmid, cosmid, phagemid, and the like. In addition, the DNA can be amplified by reverse transcriptase polymerase chain reaction (hereinafter abbreviated as RT-PCR) from total RNA or mRNA fraction prepared from the above-mentioned cells or tissues.

The DNA encoding the protein of the invention may be any DNA comprising the nucleotide sequence represented by SEQ ID NO: 4, 5, 6, 18, 19 or 20; or any DNA hybridizable to the DNA comprising the nucleotide sequence represented by SEQ ID NO: 4, 5, 6, 18, 19 or 20 under high stringent conditions and encoding a protein which has a property substantially the same as the property of the protein of the invention.

Examples of the DNA that is hybridizable to the DNA comprising the nucleotide sequence represented by SEQ ID NO: 4 under high stringent conditions include a DNA comprising a nucleotide sequence with about 99% or more, preferably about 99.5% or more homology to the nucleotide sequence represented by SEQ ID NO: 4.

Examples of the DNA that is hybridizable to the DNA comprising the nucleotide sequence represented by SEQ ID NO: 5 under high stringent conditions include a DNA comprising a nucleotide sequence with about 93% or more, preferably about 96% or more, more preferably about 99% or more homology to the nucleotide sequence represented by SEQ ID NO: 4.

Examples of the DNA that is hybridizable to the DNA comprising the nucleotide sequence represented by SEQ ID NO: 6 under high stringent conditions include a DNA comprising a nucleotide sequence with about 90% or more, preferably about 95% or more, more preferably about 97% or more homology to the nucleotide sequence represented by SEQ ID NO: 4.

Examples of the DNA that is hybridizable to the DNA comprising the nucleotide sequence represented by SEQ ID NO: 18 under high stringent conditions include a DNA comprising a nucleotide sequence with about 99.5% or more, preferably about 99.9% or more homology to the nucleotide sequence represented by SEQ ID NO: 18.

Examples of the DNA that is hybridizable to the DNA comprising the nucleotide sequence represented by SEQ ID NO: 19 under high stringent conditions include a DNA comprising a nucleotide sequence with about 93% or more, preferably about 97% or more, more preferably about 99% or more homology to the nucleotide sequence represented by SEQ ID NO: 19.

Examples of the DNA that is hybridizable to the DNA comprising the nucleotide sequence represented by SEQ ID NO: 20 under high stringent conditions include a DNA comprising a nucleotide sequence with about 85% or more, preferably about 90% or more, more preferably about 95% or more homology to the nucleotide sequence represented by SEQ ID NO: 20.

The hybridization can be carried out according to a known method or a modification thereof, for example, the method described in Molecular Cloning, 2nd Ed., J. Sambrook et al., Cold Spring Harbor Lab. Press, (1989). A commercially available library may also be used according to the instructions of the attached manufacturer's protocol. The hybridization can be carried out preferably under high stringent conditions.

The high stringent conditions used herein refer to, for example, a sodium concentration of about 19 to 40 mM, preferably about 19 to 20 mM and a temperature of about 50 to 70°C, preferably about 60 to 65°C. In particular, the hybridization condition in a sodium concentration of about 19 mM at a temperature of about 65°C is most preferred.

More specifically, the DNA encoding the protein comprising the amino acid sequence represented by SEQ ID NO: 1 includes the DNA comprising the base sequence represented by SEQ ID NO: 4; the DNA encoding the protein comprising the amino acid sequence represented by SEQ ID NO: 2 includes the DNA comprising the base sequence represented by SEQ ID NO: 5; the DNA encoding the protein comprising the amino acid sequence represented by SEQ ID NO: 3 includes the DNA comprising the base sequence represented by SEQ ID NO: 6; the DNA encoding the protein comprising the amino acid sequence represented by SEQ ID NO: 15 includes the DNA comprising the base sequence represented by SEQ ID NO: 18; the DNA encoding the protein comprising the amino acid sequence represented by SEQ ID NO: 16 includes the DNA comprising the base sequence represented by SEQ ID NO: 19; the DNA . encoding the protein comprising the amino acid sequence represented by SEQ ID NO: 17 includes the DNA comprising the base sequence represented by SEQ ID NO: 20.

The DNA encoding the partial peptide of the invention may be any DNA comprising the base sequence encoding the partial peptide of the invention described above. The DNA may be derived from any of genomic DNAs, genomic DNA library, cDNAs derived from the cells and tissues described above, cDNA library derived from the cells and tissues described above and synthetic DNAs.

The DNA encoding the partial peptide of the invention may be any DNA having a partial sequence of the DNA having the base sequence represented by SEQ ID NO: 4, 5, 6, 18, 19 or 20; or any DNA having a partial sequence of a DNA hybridizable to the DNA having the base sequence represented by SEQ ID NO: 4, 5, 6, 18, 19 or 20 under high stringent conditions and encoding a protein which has an activity substantially the same in property as the activity of the protein of the invention.

Methods for the hybridization and the high stringent conditions that can be used are the same as described above.

For cloning of the DNA completely encoding the protein of the invention or the partial peptide thereof (sometimes collectively referred to as the protein of the invention in the following description of cloning and expression of the DNA encoding the protein), the DNA may be either amplified by PCR using synthetic DNA primers containing a part of the base sequence encoding the protein of the invention, or the DNA inserted into an appropriate vector can be selected by hybridization with a labeled DNA fragment or synthetic DNA that encodes a part or the entire region of the protein of the invention. The hybridization can be carried out according to a known method, for example, the method described in Molecular Cloning, 2nd Ed., J. Sambrook et al., Cold Spring Harbor Lab. Press, (1989). A commercially available library may also be used according to the instructions of the attached manufacturer's protocol.

Substitution of the base sequence of DNA can be conducted by the PCR method or other known methods such as the ODA-LA PCR method, the Gapped duplex method or the Kunkel method, or modifications thereof using publicly known kits such as Mutant™-super Express Km (TaKaRa) or Mutan^{TM}-K (TaKaRa).

The cloned DNA encoding the protein of the invention can be used as it is, depending upon purpose or, if desired, after digestion with a restriction enzyme or after addition of a linker thereto. The DNA may contain ATG as a translation initiation codon at the 5' end and TAA, TGA or TAG as a translation termination codon at the 3' end. These translation initiation and termination codons may also be added by using an appropriate synthetic DNA adapter.

The expression vector for the protein of the invention can be produced, for example, by (a) excising the desired DNA fragment from the DNA (e.g. cDNA) encoding the protein of the invention, (b) and then ligating the DNA fragment with an appropriate expression vector downstream a promoter in the vector.

Examples of the vector include plasmids derived form E. coli (e.g. pBR322, pBR325, pUC12, pUC13), plasmids derived from Bacillus subtilis (e.g. pUB110, pTP5, pC194), plasmids derived from yeast (e.g. pSH19, pSH15), bacteriophages such as λ phage, animal viruses such as retrovirus, vaccinia virus, baculovirus, as well as pA1-11, pXT1, pRc/CMV, pRc/RSV, pcDNAI/Neo.

The promoter used in the present invention may be any promoter if it matches well with a host to be used for gene expression. In the case of using animal cells as the host, examples of the promoter include SRα promoter, SV40 promoter, HIV/LTR promoter, CMV promoter, HSV-TK promoter.

Among them, CMV (cytomegalovirus) promoter or SRα promoter is preferably used. When Escherichia bacteria are used as the host, preferred are trp promoter, lac promoter, recA promoter, λPL promoter, lpp promoter, and T7 promoter. When Bacillus bacteria are used as the host, preferred are SPO1 promoter, SPO2 promoter and penP promoter. When yeast is used as the host, preferred are PHO5 promoter, PGK promoter, GAP promoter and ADH promoter. When insect cells are used as the host, preferred are polyhedrin promoter and P 10 promoter.

In addition to the foregoing examples, the expression vector may further optionally contain an enhancer, a splicing signal, a poly A addition signal, a selection marker, or SV40 replication origin (sometimes abbreviated as SV40ori). Examples of the selection marker include dihydrofolate reductase (sometimes abbreviated as dhfr) gene [methotrexate (MTX) resistance], ampicillin resistant gene (sometimes abbreviated as Amp^{r}), and neomycin resistant gene (sometimes abbreviated as Neo^{r}, G418 resistance). In particular, when dhfr gene is used as the selection marker together with dhfr gene-deficient Chinese hamster cells, selection can also be made on thymidine-free medium.

If necessary, a signal sequence suitable for a host may be added to the N-terminal of the protein of the invention. Examples of the signal sequence that can be used are Pho A signal sequence, OmpA signal sequence for Escherichia bacteria as the host; α-amylase signal sequence, subtilisin signal sequence for Bacillus bacteria as the host; MFα signal sequence, SUC2 signal sequence for yeast as the host; and insulin signal sequence, α-interferon signal sequence, antibody molecule signal sequence for animal cells as the host, respectively.

Using the vector comprising the DNA encoding the protein of the invention thus constructed, a transformant can be produced.

Examples of the host, which may be employed, are Escherichia bacteria, Bacillus bacteria, yeasts, insect cells, insects and animal cells.

Examples of Escherichia bacteria include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)).

Examples of Bacillus bacteria include Bacillus subtilis MI114 (Gene, 24, 255 (1983)), 207-21 (Journal of Biochemistry, 95, 87 (1984)).

Examples of yeasts include Saccharomyces cereviseae AH22, AH22R-, NA87-11A, DKD-5D, 20B-12, Schizosaccharomyces pombe NCYC1913, NCYC2036, Pichia pastoris KM71.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea; and for the virus BmNPV, Bombyx mori N cell (BmN cell) is used. Examples of the Sf cell which can be used are Sf9 cell (ATCC CRL1711) and Sf21 cell (both cells are described in Vaughn, J. L. et al., In Vivo, 13, 213-217 (1977).

Examples of insects include a larva of Bombyx mori (Maeda et al., Nature, 315, 592, 1985).

Examples of animal cells include monkey cell COS-7, Vero cell, Chinese hamster cell CHO (referred to as CHO cell), dhfr gene-deficient Chinese hamster cell CHO (referred to as CHO(dhfr⁻) cell), mouse L cell, mouse AtT-20 cell, mouse myeloma cell, rat GH 3 cell, human FL cell, and H9c2 cell.

Escherichia bacteria can be transformed, for example, by the method described in Proc. Natl. Acad. Sci. U.S.A., 69, 2110 (1972), Gene, 17, 107 (1982).

Bacillus bacteria can be transformed, for example, by the method described in Molecular & General Genetics, 168, 111 (1979).

Yeasts can be transformed, for example, by the method described in Methods in Enzymology, 194, 182-187 (1991) or Proc. Natl. Acad. Sci. U.S.A., 75, 1929 (1978).

Insect cells or insects can be transformed, for example, according to the method described in Bio/Technology, 6, 47-55(1988).

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995), published by Shujunsha, or Virology, 52, 456 (1973).

Thus, the transformant, which is transformed with the expression vector comprising the DNA encoding the protein, can be obtained.

When an Escherichia or Bacillus bacterium is used as the host, the transformant can be appropriately cultured in a liquid medium which contains materials required for growth of the transformant such as carbon sources, nitrogen sources, and inorganic materials. Examples of the carbon sources include glucose, dextrin, soluble starch, and sucrose. Examples of the nitrogen sources include inorganic or organic materials such as ammonium salts, nitrate salts, corn steep liquor, peptone, casein, meat extract, soybean cake, and potato extract. Examples of the inorganic materials are calcium chloride, sodium dihydrogenphosphate, and magnesium chloride. In addition, yeast extracts, vitamins, and growth-stimulating factors may also be added to the medium. Preferably, pH of the medium is adjusted to about 5 to 8.

A preferred example of the medium for culturing Escherichia bacteria is M9 medium supplemented with glucose and casamino acids (Miller, Journal of Experiments in Molecular Genetics, 431-433, Cold Spring Harbor Laboratory, New York, 1972). If necessary, a chemical such as 3β-indolylacrylic acid can be added to the medium thereby to increase the promoter efficiency.

When an Escherichia bacterium is used as the host, the transformant is usually cultured at about 15 to 43 °C for about 3 to 24 hours. If necessary, the culture may be aerated or agitated.

When a Bacillus bacterium is used as the host, the transformant is cultured generally at about 30 to 40 °C for about 6 to 24 hours. If necessary, the culture can be aerated or agitated.

When a yeast is used as the host, the medium for culturing the transformant may be Burkholder's minimal medium (Bostian, K. L. et al., Proc. Natl. Acad. Sci. U.S.A., 77, 4505, 1980) or SD medium supplemented with 0.5% Casamino acids (Bitter, G. A. et al., Proc. Natl. Acad. Sci. U.S.A., 81, 5330, 1984). Preferably, pH of the medium is adjusted to about 5 to 8. In general, the transformant is cultured at about 20 to 35 °C for about 24 to 72 hours. If necessary, the culture can be aerated or agitated.

When an insect cell or insect is used as the host, the medium for culturing the transformant may be Grace's Insect Medium (Grace, T.C.C., Nature 195, 788 (1962)) to which an appropriate additive such as 10 % inactivated bovine serum is added. Preferably, pH of the medium is adjusted to about 6.2 to 6.4. Generally, the transformant is cultured at about 27 °C for about 3 to 5 days and, if necessary, the culture can be aerated or agitated.

When an animal cell is used as the host, the medium for culturing the transformant may be MEM medium (Science, 122, 501 (1952)), DMEM medium (Virology, 8, 396 (1959)), RPMI 1640 medium (The Journal of the American Medical Association, 199, 519 (1967)) or 199 medium (Proceeding of the Society for the Biological Medicine, 73, 1 (1950)), which contains about 5 to 20 % fetal bovine serum. Preferably, pH of the medium is adjusted to about 6 to 8. The transformant is usually cultured at about 30 to 40 °C for about 15 to 60 hours and, if necessary, the culture can be aerated or agitated.

As described above, the protein of the invention can be produced intracelluarly or extracellularly by the transformant.

The protein of the invention can be isolated or purified from the culture described above by the following procedures.

To extract the protein of the invention from the culture of bacteria or cells, after the culture is completed, the bacteria or cells are collected by a well known method and suspended in an appropriate buffer. The bacteria or cells are disrupted by a well known method such as ultrasonication, treatment with lysozyme or freeze-thaw cycling, and then subjected to the centrifugation or filtration to obtain the crude protein extract. The buffer may contain a protein denaturing agent such as urea or guanidine hydrochloride, or a surfactant such as Triton X-100™. When the protein of the invention is secreted into the culture broth, after the culture is completed, the supernatant can be separated and collected from the bacteria or cells by a well known method.

The protein of the invention contained in the supernatant or the extract thus obtained can be purified by an appropriate combination of well-known isolation or purification methods. Such isolation or purification methods include a method utilizing difference in solubility such as salting out, solvent precipitation; a method mainly utilizing difference in molecular weight such as dialysis, ultrafiltration, gel filtration, SDS-polyacrylamide gel electrophoresis; a method utilizing difference in electric charge such as ion exchange chromatography; a method utilizing specific affinity such as affinity chromatography; a method utilizing difference in hydrophobicity such as reversed phase high performance liquid chromatography; a method utilizing difference in isoelectric point such as isoelectric focusing; and the like.

When the protein of the invention thus obtained is in a free form, it can be converted into a salt form by a well known method or a modification thereof. On the other hand, when the protein is obtained in a salt form; it can be converted into the free form or a different salt form by a well known method or a modification thereof.

The protein of the invention produced by the recombinant can be treated, before or after the purification, with an appropriate protein-modifying enzyme so that the protein can be appropriately modified or deprived of a partial polypeptide. Examples of the protein-modifying enzyme include trypsin, chymotrypsin, arginyl endopeptidase, protein kinase, glycosidase and the like.

The thus produced protein of the invention can be detected using a specific antibody by an enzyme immunoassay or western blotting method.

The antibody to the protein of the invention, the partial peptide or the salt thereof may be any polyclonal antibodies or monoclonal antibodies, which are capable of recognizing the protein of the invention, the partial peptide or the salt thereof.

The antibody to the protein of the invention, the partial peptide or the salt thereof (occasionally referred to simply as the protein of the invention in the description of antibodies) can be produced using the protein of the invention as an antigen by a known production method for antibodies or antisera.

### [Preparation of monoclonal antibody]

### (a) Preparation of monoclonal antibody-producing cells

The protein of the invention is administered to warm-blooded animals either alone or together with carriers or diluents to the site which can induce the antibody production. To potentiate the antibody productivity, complete Freund's adjuvants or incomplete Freund's adjuvants may be administered at the same time. The administration is usually carried out once every 2 to 6 weeks and 2 to 10 times in total. Examples of applicable warm-blooded animals are monkeys, rabbits, dogs, guinea pigs, mice, rats, sheep, goats and chickens, with mice and rats being preferred.

To prepare monoclonal antibody-producing cells, a warm-blooded animal, e.g. mouse is immunized with an antigen, an individual whose antibody titer is high is selected, and then its spleen or lymph node is excised after 2 to 5 days from the final immunization. Antibody-producing cells contained therein are fused with myeloma cells of the same or different type animal to give a monoclonal antibody-producing hybridoma. The antibody titer in antisera may be assayed by reacting the labeled protein as described later, with the antiserum, and assaying the activity of the label bound to the antibody. The cell fusion may be carried out according to the known method by Koehler and Milstein (Nature, 256, 495 (1975)). Afusion promoter such as polyethylene glycol (PEG) and Sendai virus, preferably PEG, may be used.

The myeloma cells are ones derived from warm-blooded animals, such as NS-1, P3U1, SP2/0, AP-1. In particular, P3U1 is preferably employed. A preferred count ratio of the antibody-producing cells (spleen cells) to the myeloma cells is within a range of about 1:1 to 20:1. To efficiently carry out the cell fusion, it is preferred to add PEG (preferably PEG 1000 to PEG 6000) in a concentration of about 10 to 80 % and incubate at 20 to 40 °C, preferably at 30 to 37 °C for 1 to 10 minutes.

There are various methods used for screening of a monoclonal antibody-producing hybridoma. For example, a method may be used, which comprises adding the supernatant of hybridoma to a solid phase (e.g. microplate) adsorbed with the protein antigen directly or together with a carrier; adding an anti-immunoglobulin antibody (when mouse cells are used for the cell fusion, anti-mouse immunoglobulin antibody is used) which is labeled with a radioactive substance or an enzyme, or adding Protein A; and detecting the monoclonal antibody bound to the solid phase. Another method may be used, which comprises adding the supernatant of hybridoma to a solid phase adsorbed with an anti-immunoglobulin antibody or Protein A; adding the protein labeled with a radioactive substance or an enzyme; and detecting the monoclonal antibody bound to the solid phase.

The monoclonal antibody can be selected according to a publicly known method or its modification. In general, the selection can be carried out in a medium for animal cells supplemented with HAT (hypoxanthine, aminopterin and thymidine). Any medium can be used for the selection and growth as long as the hybridoma can grow there. For example, RPMI 1640 medium containing 1 to 20 %, preferably 10 to 20 % fetal bovine serum, GIT medium (Wako Pure Chemical Industries) containing 1 to 10 % fetal bovine serum, or a serum free medium for hybridoma culture (SFM-101, Nissui Seiyaku) can be used. In general, the hybridoma may be cultured at 20 to 40 °C, preferably at 37 °C for about 5 days to about 3 weeks, preferably 1 to 2 weeks under 5 % CO₂. The antibody titer in the supernatant of the hybridoma culture can be determined in the same way as described above for the antibody titer in antisera.

### (b) Purification of monoclonal antibody

The monoclonal antibody may be isolated or purified according to a publicly known method, e.g. the method for isolating or purifying immunoglobulins (for example, salting-out, alcohol precipitation, isoelectric point precipitation, electrophoresis, adsorption and desorption with ion exchangers (e.g. DEAE), ultracentrifugation, gel filtration, or a specific purification method which comprises collecting only an antibody with an activated adsorbent such as an antigen-binding solid phase, Protein A or Protein G, and dissociating the binding to obtain the antibody).

### [Preparation of polyclonal antibody]

The polyclonal antibody of the invention can be produced by publicly known methods or modifications thereof. For example, a warm-blooded animal is immunized with an immunogen (a protein antigen) per se, or a complex of an immunogen and a carrier protein in a manner similar to the method described above for the production of monoclonal antibodies. The product containing the antibody to the protein of the invention is collected from the immunized animal, followed by separation and purification of the antibody.

In the complex of immunogen and carrier protein used to immunize a warm-blooded animal, any type of carrier protein and any mixing ratio of carrier to hapten can be used as long as the antibody is efficiently produced to the immunized hapten crosslinked to the carrier. For example, bovine serum albumin, bovine thyroglobulin or hemocyanin is crosslinked to the hapten in a carrier/hapten weight ratio of about 0.1 to 20, preferably about 1 to 5.

A variety of condensation agents can be used for the coupling of carrier to hapten. Glutaraldehyde, carbodiimide, maleimide-activated ester, activated ester reagents containing thiol group or dithiopyridyl group, and others are used for the coupling.

The condensate is administered to warm-blooded animals either alone or together with carriers or diluents to the site which can induce the antibody production. At the same time, to potentiate the antibody productivity, complete Freund's adjuvant or incomplete Freund's adjuvant may be administered. The administration is usually made once every about 2 to 6 weeks about 3 to 10 times in total.

The polyclonal antibody can be collected from the blood or ascites, preferably from the blood of warm-blooded animals immunized by the method described above.

The polyclonal antibody titer in antiserum can be assayed in the same way as described above for the assay of serum antibody titer. As well as the monoclonal antibody, the polyclonal antibody can also be isolated or purified according to the immunoglobulin purification method.

The antisense nucleotide having a base sequence complementary or substantially complementary to the DNA encoding the protein or the partial peptide of the invention (this DNA may be referred to as the DNA of the invention in the following description of the antisense nucleotide) refers to any antisense nucleotide having a base sequence complementary or substantially complementary to the DNA of the invention and having an activity to inhibit the expression of the DNA; the antisense DNA is preferred.

The base sequence substantially complementary to the DNA of the invention includes base sequences having at least about 97%, preferably at least about 98%, more preferably at least about 99% homology to the whole or a part of the base sequence complementary to the DNA of the invention (i.e. a complementary strand of the DNA of the invention). In particular, preferred is an antisense nucleotide having at least about 97%, preferably at least about 98%, more preferably at least about 99% homology to the complementary strand of the partial base sequence encoding the N-terminal region of the protein of the invention (e.g. the base sequence around the initiation codon) in the whole base sequence of the complementary strand of the DNA of the invention,

The antisense nucleotide may consist of usually about 10 to 40 and preferably 15 to 30 bases.

To prevent the degradation by hydrolase such as nuclease, phosphate residues (phosphates) of respective nucleotides constituting the antisense nucleotide may be replaced with chemically modified phosphate residues such as phosphorothioate, methyl phosphonate, and phosphorodithionate. These antisense nucleotides can be produced using a well known DNA synthesizer.

Described below are utilities of the protein of the invention, the partial peptide or the salt thereof (occasionally referred to as the protein of the invention); the DNA encoding the protein of the invention or the partial peptide thereof (occasionally referred to as the DNA of the invention); the antibody to the protein of the invention or the partial peptide or the salt thereof (occasionally referred to as the antibody of the invention); and the antisense nucleotide to the DNA of the invention (occasionally referred to as the antisense nucleotide of the invention).

A pharmaceutical composition comprising a compound or a salt thereof regulating the activity of the protein of the invention is useful as a prophylactic and/or therapeutic agent for heart diseases (e.g. heart failure after cardiac infarction; angina pectoris; myocardosis; heart failure derived from angina pectoris and myocardosis, etc), central nervous system diseases (e.g. neurodegenerative diseases (e.g. Alzheimer's disease, Parkinson's disease, amyotrophic lateral sclerosis (ALS), Huntington's disease, spinocerebellar degeneration), neuropsychiatric disorder (e.g. schizophrenia), head injury, spinal cord injury, cerebrovascular disorder (e.g. cerebral infarction, cerebral edema), cerebrovascular dementia, etc), and the like.

### [1] Screening for a pharmaceutical candidate compound for diseases

The protein of the invention is considered to play an important role in the maintenance of the heart functions and the central nervous functions. Therefore, a compound or a salt thereof regulating the activity of the protein can be used as a prophylactic and/or therapeutic agent for heart diseases (e.g. heart failure after cardiac infarction; angina pectoris; myocardosis; heart failure derived from angina pectoris and myocardosis, etc), central nervous system diseases (e.g. neurodegenerative diseases (e.g. Alzheimer's disease, Parkinson's disease, amyotrophic lateral sclerosis (ALS), Huntington's disease, spinocerebellar degeneration), neuropsychiatric disorder (e.g. schizophrenia), head injury, spinal cord injury, cerebrovascular disorder (e.g. cerebral infarction, cerebral edema), cerebrovascular dementia, etc), and the like.

Accordingly, the protein of the invention is useful as a reagent for screening for a compound or a salt thereof regulating the activity of the protein of the invention.

In this way, the present invention provides a method of screening for a compound or a salt thereof regulating the activity of the protein of the invention, which comprises using the protein of the invention (sometimes referred to as the screening method of the invention).

Examples of the screening method of the invention are described below.

### 1. Screening methods using the gene expression as an indicator

(1-1) The following cases (i) and (ii) are compared to thereby select compounds which regulate the activity of the protein of the invention or which regulate the expression of the protein gene of the invention:
   (i) a cell having the ability to produce the protein of the invention is cultured under stresses, preferably under such conditions as nutrient starvation, low oxygen; and
   (ii) a cell having the ability to produce the protein of the invention is cultured in a mixture with a test compound under stresses, preferably under such conditions as nutrient starvation, low oxygen.
(1-2) The following cases (i) and (ii) are compared to thereby select compounds which regulate the activity of the protein of the invention or which regulate the expression of the protein gene of the invention:
   (i) a cell into which a plasmid comprising a reporter gene (e.g. luciferase gene) linked to the promoter of the DNA of the invention is introduced is cultured under stresses, preferably under such conditions as nutrient starvation, low oxygen, etc. and
   (ii) a cell into which a plasmid comprising a reporter gene (e.g. luciferase gene) linked to the promoter of the DNA of the invention is introduced is cultured in a mixture with a test compound under stresses, preferably under such conditions as nutrient starvation, low oxygen.

For the methods (1-1) and (1-2) above, for example, the gene expression levels of the protein of the invention (specifically, amounts of the protein or amounts of mRNA encoding the protein) in (i) and (ii) are measured and compared.

For example, if a test compound inhibits or promotes the gene expression level in the above-mentioned cell by about 20% or more, preferably by about 30% or more, more preferably by about 50% or more, compared to the gene expression level in the absence of the test compound, the test compound can be selected as a compound that inhibits or promotes the activity of the protein of the invention or as a compound that inhibits or promotes the gene expression of the protein of the invention.

Gene expression levels may be measured by conventional methods such as RT-PCR, real time PCR analysis system (ABI; TaqMan polymerase chain reaction), reporter gene assay, ELISA, and similar methods.

The term "low oxygen condition" used herein means conditions where the oxygen concentration is, for example, 20% or less, e.g. 2% (Nature 394: 485-490, 1998). The term "nutrient starvation" means to culture both in the absence of serum and in the presence of 2-deoxyglucose.

In a preferred embodiment, (a) the test compound which promotes the gene expression of a protein comprising an amino acid sequence identical with or substantially identical with the amino acid sequence as shown in SEQ ID NO: 1 or 15 (e.g. mRNA encoding the protein) or (b) the test compound which inhibits the gene expression of a protein comprising an amino acid sequence identical with or substantially identical with the amino acid sequence as shown in SEQ ID NO: 2, 3, 16 or 17, (e.g. mRNA encoding the protein) is selected.

### 2. Screening methods using the gene expression controlled by the protein of the invention as an indicator

The protein of the invention controls the expression of heart-specific and central nerve cell-specific genes to thereby play an important role in the maintenance of functions of the heart and the central nerve system. Therefore, by a screening method using the expression of a heart-specific or central nerve cell-specific gene controlled by the protein of the invention as an indicator, it is possible to obtain compounds or salts thereof that regulate the activity of the protein of the invention.
(2-1) The following cases (i) and (ii) are compared:
   (i) a cell in which the expression of a gene controlled by the protein of the invention (e.g. atrial natriuretic peptide gene, α myosin heavy chain gene, N-methyl-D-aspartate receptor gene, or the like) is recognized is cultured under stresses, preferably under such conditions as nutrient starvation, low oxygen; and
   (ii) a cell in which the expression of a gene controlled by the protein of the invention (e.g. atrial natriuretic peptide gene, α myosin heavy chain gene, N-methyl-D-aspartate receptor gene, or the like) is recognized is cultured in a mixture with a test compound under stresses, preferably under such conditions as nutrient starvation, low oxygen.
(2-2) The following cases (i) and (ii) are compared:
   (i) a cell into which a plasmid comprising a reporter gene (e.g. luciferase gene) linked to the promoter of the gene controlled by the protein of the invention is introduced is cultured under stresses, preferably under such conditions as nutrient starvation, low oxygen; and
   (ii) a cell into which a plasmid comprising a reporter gene (e.g. luciferase gene) linked to the promoter of the gene controlled by the protein of the invention is introduced is cultured in a mixture with a test compound under stresses, preferably under such conditions as nutrient starvation, low oxygen.
(2-3) The following cases (i) and (ii) are compared:
   (i) a cell into which the DNA of the invention and a DNA encoding a protein that interacts with the protein of the invention (e.g. GATA-4, MASH-1, Nkx2.5/Csx, Sp-1, or the like) are introduced is cultured under stresses, preferably under such conditions as nutrient starvation, low oxygen; and
   (ii) in a mixture with a) a test compound, b) a cell into which the DNA of the invention and a DNA encoding a protein that interacts with the protein of the invention are introduced is cultured under stresses, preferably under such conditions as nutrient starvation, low oxygen.

For the methods (2-1) to (2-3) above, for example, the expression levels of a gene controlled by the protein of the invention in (i) and (ii) are measured and compared.

For example, if a test compound inhibits or promotes the gene expression level in the above-mentioned cell by about 20% or more, preferably by about 30% or more, more preferably by about 50% or more, compared to the gene expression level in the absence of the test compound, the test compound can be selected as a compound that inhibits or promotes the activity of the protein of the invention.

Gene expression levels may be measured in the same manner as described in Section 1 above. The term "low oxygen condition" has the same meaning as defined in Section 1 above.

### 3. Screening methods using the binding activity between the protein of the invention and a protein that interacts therewith as an indicator

It is known that the protein of the invention interacts with proteins playing important roles in the maintenance of functions of the heart and the central nerve system [e.g. GATA-4 (cell-specific transcription factor; The EMBO Journal, 19: 2046-2055, 2000), MASH-1 (nerve-specific transcription factor; FEBS Letters, 472: 53-56, 2000), Nx2.5/Csx (heart-specific transcription factor; Journal of Biological Chemistry, 273: 34904-34910, 1998) and the like]. Therefore, by screening methods using the binding activity between the protein of the invention and a protein that interacts therewith as an indicator, it is possible to obtain compounds, or salts thereof, that regulate the activity of the protein of the invention.

Binding activities between the protein of the invention and a protein that interacts therewith in the following cases (i) and (ii) are compared:
(i) a) the protein of the invention and b) a protein that interacts therewith (e.g. GATA-4, MASH-1, Nkx2.5/Csx, Sp-1 or the like) are contacted; and
(ii) a) a test compound, b) the protein of the invention and c) a protein that interacts with the protein of the invention are contacted.

The protein that interacts with the protein of the invention may be either novel or known.

Binding activities may be measured according to conventional methods such as the yeast or mammal two hybrid method, SPA, ELISA, etc.

In a specific example using the yeast or mammal two hybrid method, the following cases (i) and (ii) are compared:
(i) a cell into which a) a plasmid that expresses a fusion protein composed of the protein of the invention and the DNA binding domain of GAL4 gene, b) a plasmid that expresses a fusion protein composed of a protein that interacts with the protein of the invention and the transcription activation domain of VP16 gene, and c) a plasmid comprising a reporter gene (e.g. luciferase gene) linked to the promoter of GAL4 gene is introduced is cultured alone; and
(ii) the cell described in (1) above is cultured in the presence of a test compound.

In the above-described method, for example, expression levels of the reporter gene in (i) and (ii) are measured and compared. The cell used therein may be any cell and may be cultured in a medium suitable for the screening.

The protein of the invention used in SPA or ELISA assays may be labeled with a radio isotope such as [¹²⁵I], [¹³¹I], [³H], [¹⁴C] or the like, or may be a fusion protein with GST.

For example, in the above-described screening method, if a test compound inhibits or promotes the binding activity by about 20% or more, preferably by about 30% or more, more preferably by about 50% or more, compared to the binding activity in the absence of the test compound, the test compound can be selected as a compound that inhibits or promotes the activity of the protein of the invention.

Examples of test compounds, which may be used in the above-mentioned screening methods 1 to 3 include peptides, proteins, biological non-peptide compounds (e.g. sugars, lipids), synthetic compounds, microorganism cultures, cell extracts, plant extracts, and animal tissue extracts; these compounds may be novel or publicly known.

Examples of the cells (1) to (5) above include primary myocardial cells or central nerve cells having the ability to produce the protein of the invention and host cells (transformants) transformed with the above-mentioned vector comprising the DNA encoding the protein of the invention. As the host, an animal cell such as H9c2 cell (ATCC No. CRL-1446), P 19 cell (ATCC No. CRL-1825) and the like may be preferably used. In the above-described screening methods, for example, the transformant which is cultured by the methods as described above to express intracellularly the protein of the invention may be preferably used.

The compounds or salts thereof that inhibit the activity of the protein of the invention (inhibitors) or promote the activity of the protein of the invention (promoting agents), selected by the screening methods of the invention described above, have an effect of protecting myocardial cells and central nerve cells, as well as an action of inhibiting myocardial cell death and central nerve cell death.

At the time of cell death or cell injury, a moderate promotion of the activity of the gene comprising the DNA of the invention is expected to protect myocardial cells and central nerve cells (the function-protecting effect). Further, it is considered that an excessive promotion of the activity of the gene comprising the DNA of the invention causes the excessive activation of cells to thereby accelerate the exhaustion of the cells. Therefore, it is important to control the said activity properly. For example, at the time of loss of myocardial cells or central nerve cells, if it is considered that cell injury is caused by promotion of the activity of the gene, the compound or a salt thereof that inhibits the activity of the protein of the invention (inhibitor) is administered; if it is considered that cell injury is caused by decrease in the activity of the gene, a compound or a salt thereof that promotes the activity of the protein of the invention (promoting agent) is administered.

The cell-protecting effect can be expressed by the activation rate or survival rate of myocardial cells or central nervous cells. Specifically, the rates can be determined by generalized methods such as the MTT (3-(4,5-Dimethyl-2-thiazolyl)-2,5-diphenyl-2H-tetrazolium) method for measurement of respiratory activity, the trypan blue staining method, or the TUNNEL staining method (Terminal deoxytransferase-mediated dUTP-X nick end labeling, Cell 97: 189-198, 1999).

The screening kit of the invention comprises the protein of the invention or the cell having the ability of producing the protein of the invention.

The compound or a salt thereof, which is obtained by the screening method or the screening kit of the invention, is selected from the above described test compounds such as peptides, proteins, biological non-peptide compounds (e.g. sugars, lipids), synthetic compounds, microorganism cultures, fermentation products, cell extracts, plant extracts, animal tissue extracts, and blood plasma, and can regulate the activity of the protein of the invention (the compound enhancing or inhibiting the activity of the protein of the invention).

The salt of the compound, which may be used, includes the same types as the above-described salts of the protein of the invention.

The compound or salt thereof regulating (enhancing or inhibiting) the activity of the protein of the invention shows the inhibitory effect on death of myocardial cells and central nervous cells, and thus is useful as a prophylactic and/or therapeutic agent for heart diseases (e.g. heart failure after cardiac infarction; angina pectoris; myocardosis; heart failure derived from angina pectoris and myocardosis, etc), central nervous system diseases (e.g. neurodegenerative diseases (e.g. Alzheimer's disease, Parkinson's disease, amyotrophic lateral sclerosis (ALS), Huntington's disease, spinocerebellar degeneration), neuropsychiatric disorder (e.g. schizophrenia), head injury, spinal cord injury, cerebrovascular disorder (e.g. cerebral infarction, cerebral edema), cerebrovascular dementia, etc), and the like.

When the compound or salt thereof, which is obtained by the screening method or the screening kit of the invention, is used as the above-described prophylactic and/or therapeutic agent, it can be formulated into a pharmaceutical composition in a conventional manner.

Since the thus obtained pharmaceutical composition is safe and low toxic, it can be administered orally or parenterally to a human or non-human warm-blooded animal (e.g. mouse, rat, rabbit, sheep, swine, bovine, horse, fowl, cat, dog, monkey, chimpanzee).

The composition for oral administration is exemplified by a solid or liquid drug form, specifically a tablet (including sugar-coated and film-coated table), pill, granule, powder, capsule (including soft capsule), syrup, emulsion, suspension. Such a composition is prepared by a publicly known method and contains a carrier, diluting agent or vehicle generally used in the pharmaceutical field. Examples of the carrier or vehicle used for the tablet include lactose, starch, sucrose, magnesium stearate.

Examples of the composition used for parenteral administration includes an injection and a suppository. The injection includes such forms as intravenous injection, subcutaneous injection, endodermic injection, intramuscular injection, drop injection. Such injections may be prepared by dissolving, suspending or emulsifying the antibody or its salt as described above in sterile aqueous or oily liquid generally used for an injection in accordance with a publicly known method. Examples of an aqueous liquid for injection include a physiological saline and an isotonic solution containing glucose and other auxiliary agents, which may be used in combination with an appropriate dissolution aid such as an alcohol (e.g. ethanol), polyalcohol (e.g. propylene glycol, polyethylene glycol), nonionic surfactant (e.g. polysorbate 80^{TM}, HCO-50 (polyoxyethylene (50 mol) adduct of hydrogenated castor oil)). Examples of the oily liquid include sesame oil and soybean oil, which may be used in combination with a dissolution aid such as benzyl benzoate and benzyl alcohol. The injection prepared is usually filled in an appropriate ampoule. The suppository used for administration into rectum is prepared by compounding the antibody or its salt as described above with a normal base material for a suppository.

It is advantageous to prepare the above-described pharmaceutical composition for oral or parenteral use in a unit dosage form containing a suitable dose of the active component. The unit dosage form is exemplified by a table, pill, capsule, injection (ampoule), suppository. It is preferable that the unit dosage form normally contains 5 to 500 mg: particularly 5 to 100 mg for the injection and 10 to 250 mg for other forms.

The dose of the compound or its salt varies depending on its effect, a target disease, a subject, an administration route, etc. For example, when administering orally the compound or its salt to an adult (60 kg body weight) for treatment of heart failure, the daily dose is normally about 0.1 to 100 mg, preferably about 1.0 to 50 mg, and more preferably about 1.0 to 20 mg of the compound or its salt. Also in parenteral administration, the single dose of the compound or its salt varies depending on a subj ect, a target disease, etc. For example, when administering the compound or its salt to an adult (60 kg body weight) for treatment of heart failure in an injection form, it is advantageous to inject intravenously about 0.01 to 30 mg, preferably about 0.1 to 20 mg, and more preferably about 0.1 to 10 mg of the compound or its salt per day. For other animal species, the corresponding dose as converted per 60 kg body weight can be administered.

### [2] Quantification of the protein of the invention, the partial peptide or the salt thereof

The antibody of the invention is capable of specifically recognizing the protein of the invention, and thus can be used for a quantification of the protein of the invention in a test liquid sample, in particular, for the quantification by sandwich immunoassay.

Thus, the present invention provides:
(i) a method of quantifying the protein of the invention in a test liquid sample, which comprises competitively reacting the antibody of the invention with the test liquid sample and a labeled form of the protein of the invention, and measuring the ratio of the labeled protein bound to the antibody; and
(ii) a method of quantifying the protein of the invention in a test liquid sample, which comprises reacting the test liquid sample simultaneously or sequentially with the antibody of the invention immobilized on a carrier and a labeled form of another antibody of the invention, and then measuring the activity of the label on the immobilizing carrier.

In the quantification method (ii) described above, it is preferred that one antibody is capable of recognizing the N-terminal region of the protein of the invention, and the other antibody is capable of reacting the C-terminal region of the protein of the invention.

The monoclonal antibody to the protein of the invention (hereinafter sometimes referred to as the monoclonal antibody of the invention) can be used to quantify the protein of the invention, and to detect the protein in a tissue staining method as well. For these purposes, the antibody molecule per se may be used, or F(ab')₂, Fab' or Fab fraction of the antibody molecule may also be used.

There is no particular limitation for the type of quantification method using the antibody to the protein of the invention, and any assay methods can be used whereby the amount of antibody, antigen, or antibody-antigen complex corresponding to the amount of antigen (e.g. the amount of the protein) in a test liquid sample can be detected by chemical or physical means and the amount of the antigen can be calculated from a standard curve prepared from standard solutions containing known amounts of the antigen. Advantageously used are, for example, nephrometry, competitive method, immunometric method and sandwich method. In terms of sensitivity and specificity, the sandwich method, as described below, is particularly preferred.

Examples of the labeling agent used in the assay method using the labeled substance include radioisotopes, enzymes, fluorescent substances and luminescent substances, etc. Examples of the radioisotope include [¹²⁵I], [¹³¹I], [³H], and [¹⁴C]. Preferred examples of the enzyme are ones that are stable and have a high specific activity, including β-galactosidase, β-glucosidase, alkaline phosphatase, peroxidase, and malate dehydrogenase. Examples of the fluorescent substance include fluorescamine and fluorescein isothiocyanate. Examples of the luminescent substance include luminol, luminol derivatives, luciferin, and lucigenin. Furthermore, the biotin-avidin system may also be used for coupling of an antibody or antigen to the labeling agent.

For the immobilization of antigens or antibodies, physical attachment may be used. Alternatively, chemical coupling that is conventionally used for immobilization of proteins or enzymes may be used as well. Examples of the carrier include insoluble polysaccharides such as agarose, dextran and cellulose; synthetic resins such as polystyrene, polyacrylamide, silicone; or glass.

In the sandwich method, a test liquid sample is reacted with an immobilized monoclonal antibody of the invention (primary reaction), then reacted with another labeled monoclonal antibody of the invention (secondary reaction) and the activity of the label on the immobilizing carrier is assayed, whereby the amount of the protein of the invention in the test liquid sample can be quantified. The primary and secondary reactions may be carried out in a reversed order, simultaneously or sequentially with an interval. The type of the labeling agent and the method for the immobilization may be the same as those described above. In the immunoassay by the sandwich method, it is not always necessary that one type of the antibody is used for the immobilized or labeled antibody, but a mixture of two or more antibodies may also be used for the purpose of improving the measurement sensitivity.

In the quantification of the protein of the invention by the sandwich method, it is preferred that the monoclonal antibodies of the invention used for the primary and secondary reactions have different binding sites on the protein of the invention, respectively. Thus, in respect to the antibodies used in the primary and secondary reactions, for example, when the antibody used in the secondary reaction recognizes the C-terminal region of the protein of the invention, the antibody used in the primary reaction preferably recognizes a region other than the C-terminal region, for example, the N-terminal region.

In the competitive method, an antigen in a test liquid sample and a labeled antigen are competitively reacted with an antibody, and then the unreacted labeled antigen (F) and the labeled antigen bound to the antibody (B) are separated (i.e. B/F separation), and the amount of the label present in either B or F is measured to determine the amount of the antigen in the test liquid sample. For this reaction, used are a liquid phase method in which the used antibody is soluble and the B/F separation is performed by polyethylene glycol and a second antibody to the said antibody; and a solid phase method in which the first antibody is an immobilized one, or the first antibody is soluble and the second antibody is an immobilized one.

In the immunometric method, an antigen in a test liquid sample and an immobilized antigen are competitively reacted with a given amount of the labeled antibody, and then the solid phase is separated from the liquid phase; or an antigen in a test liquid sample and an excess amount of the labeled antibody are reacted, and then an immobilized antigen is added to bind the unreacted labeled antibody to the solid phase and the solid phase is separated from the liquid phase. Subsequently, the amount of the label in either of the phases is measured to determine the antigen amount in the test liquid sample.

In the nephrometry, the amount of insoluble sediment, which is produced as a result of the antigen-antibody reaction in a gel or in a solution, is measured. Even when the amount of an antigen in a test liquid sample is small and only a small amount of the sediment is obtained, a laser nephrometry utilizing laser scattering can be suitably used.

For applying these immunological methods to the quantification method of the invention, any special conditions or procedures are not required. A system for quantifying the protein of the invention may be constructed according to the combination of the usual technical consideration in the art and the conventional conditions and procedures. For the details of these general techniques, reference can be made to any reviews and textbooks.

See, for example, Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immunochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies))(all published by Academic Press).

As described above, the protein of the invention can be quantified with high sensitivity, using the antibody of the invention.

Furthermore, (1) when a decreased level of the protein of the invention is detected in a subject by quantifying the protein level using the antibody of the invention, the subject may be diagnosed as having or likely to have in the future, for example, heart diseases (e.g. heart failure after cardiac infarction; angina pectoris; myocardosis; heart failure derived from angina pectoris and myocardosis, etc) or central nervous system diseases (e.g. neurodegenerative diseases (e.g. Alzheimer's disease, Parkinson's disease, amyotrophic lateral sclerosis (ALS), Huntington's disease, spinocerebellar degeneration), neuropsychiatric disorder (e.g. schizophrenia), head injury, spinal cord injury, cerebrovascular disorder (e.g. cerebral infarction, cerebral edema), cerebrovascular dementia, etc).

The antibody of the invention can be used for detecting the protein of the invention in a test sample such as a body fluid and a tissue. The antibody can also be used for preparation of an antibody column for purification of the protein of the invention, detection of the protein in fractions upon purification, and analysis of the behavior of the protein in cells under investigation.

### [3] Genetic diagnosis agents

The DNA of the invention can be used as a probe, for example, to detect an abnormality of the DNA or mRNA encoding the protein of the invention or the partial peptide thereof in a human or non-human warm-blooded animals (e.g. rat, mouse, guinea pig, rabbit, fowl, sheep, swine, bovine, horse, cat, dog, monkey, chimpanzee)(gene abnormality). Therefore, it is useful as a genetic diagnosis agent for detecting the damage, mutation, decreased expression, or increased expression or overexpression of said DNA or mRNA.

The genetic diagnosis described above using the DNA of the invention can be performed by, for example, the well known northern hybridization assay or the PCR-SSCP assay (Genomics, 5, 874-879 (1989); Proceedings of the National Academy of Sciences of the United States of America, 86, 2766-2770 (1989)), etc.

For example, when an overexpression of the DNA is detected in a subject by the northern hybridization assay or when a mutation of the DNA is detected by the PCR-SSCP assay, the subject can be diagnosed as highly likely to have, for example, heart diseases (e.g. heart failure after cardiac infarction; angina pectoris; myocardosis; heart failure derived from angina pectoris and myocardosis, etc) or central nervous system diseases (e.g. neurodegenerative diseases (e.g. Alzheimer's disease, Parkinson's disease, amyotrophic lateral sclerosis (ALS), Huntington's disease, spinocerebellar degeneration), neuropsychiatric disorder (e.g. schizophrenia), head injury, spinal cord injury, cerebrovascular disorder (e.g. cerebral infarction, cerebral edema), cerebrovascular dementia, etc).

### [4] Pharmaceuticals containing the antisense nucleotide

The antisense nucleotide of the invention capable of binding complementally to the DNA of the invention and of suppressing the expression of the DNA shows low toxicity and can inhibit in vivo the functions of the protein or the DNA of the invention (e.g. the activity of enhancing cardiac hypofunction). Therefore, it can be used as a prophylactic and/or therapeutic agent for, for example, heart diseases (e.g. heart failure after cardiac infarction; angina pectoris; myocardosis; heart failure derived from angina pectoris and myocardosis, etc), central nervous system diseases (e.g. neurodegenerative diseases (e.g. Alzheimer's disease, Parkinson's disease, amyotrophic lateral sclerosis (ALS), Huntington's disease, spinocerebellar degeneration), neuropsychiatric disorder (e.g. schizophrenia), head injury, spinal cord injury, cerebrovascular disorder (e.g. cerebral infarction, cerebral edema), cerebrovascular dementia, etc), and the like.

When used as the prophylactic and therapeutic agent as described above, the said antisense nucleotide can be formulated and administered according to a well-known method.

For example, when used, the antisense nucleotide can be orally or parenterally administered to a human or non-human warm-blooded animals (e.g. mouse, rat, rabbit, sheep, swine, bovine, horse, fowl, cat, dog, monkey, chimpanzee) in a conventional manner, alone or after inserted into a suitable vector such as retrovirus vector, adenovirus vector, or adenovirus-associated virus vector. The antisense nucleotide can be administered using a gene gun or such a catheter as a hydrogel catheter, as it is or as formulated with a physiologically acceptable carrier such as an auxiliary material for uptake enhancement.

The dose of the antisense nucleotide varies depending on a target disease, a subject, an administration route, etc. For example, when the antisense nucleotide of the invention is orally administered for treatment of heart failure, the daily dose for an adult (60 kg body weight) is about 0.1 to 100 mg.

Further, the antisense nucleotide can be used as a diagnostic oligonucleotide probe to examine the existence or expression profile of the DNA of the invention in a tissue or a cell.

### [5] Pharmaceuticals containing the antibody of the invention

The antibody of the invention having an effect to neutralize the activity of the protein of the invention can be used as a prophylactic and/or therapeutic agent for heart diseases (e.g. heart failure after cardiac infarction; angina pectoris; myocardosis; heart failure derived from angina pectoris and myocardosis, etc), central nervous system diseases (e.g. neurodegenerative diseases (e.g. Alzheimer's disease, Parkinson's disease, amyotrophic lateral sclerosis (ALS), Huntington's disease, spinocerebellar degeneration), neuropsychiatric disorder (e.g. schizophrenia), head injury, spinal cord injury, cerebrovascular disorder (e.g. cerebral infarction, cerebral edema), cerebrovascular dementia, etc), and the like.

The prophylactic and therapeutic agent containing the antibody of the invention for the aforementioned diseases are low toxic and can be orally or parenterally administered to a human and non-human warm-blooded animals (e.g. mouse, rat, rabbit, sheep, swine, bovine, horses, fowl, feline, canine, monkey, chimpanzee) as an intact solution or a pharmaceutical composition in a proper form.

The dose varies depending on a subject to be administered to, a target disease, a symptom, a route for administration, etc. For example, when used for prevention or treatment of heart failure in an adult, it is preferable that the antibody of the invention is intravenously administered normally in a single dose of about 0.01 to 20 mg/ kg body weight, preferably about 0.1 to 10 mg/ kg body weight, and more preferably about 0.1 to 5 mg/ kg body weight at 1 to 5 times a day, and preferably 1 to 3 times a day. A dose based on those given above can be administered in another parenteral administration and an oral administration. When the symptom is very severe, the dose may be increased depending on the symptom.

The antibody of the invention can be administered as it is or as a proper pharmaceutical composition. The pharmaceutical composition used for the above-described administration contains the antibody of the invention or its salt with a physiologically acceptable carrier, a diluting agent, or a vehicle. Such a composition is prepared in an orally or parentally suitable drug form.

Thus, the composition for oral administration is exemplified by a solid or liquid drug form, specifically a tablet (including sugar-coated and film-coated table), pill, granule, powder, capsule (including soft capsule), syrup, emulsion, suspension. Such a composition is prepared by a publicly known method and contains a carrier, diluting agent or vehicle generally used in the pharmaceutical field. Examples of the carrier or vehicle used for the tablet include lactose, starch, sucrose, magnesium stearate.

Examples of the composition used for parenteral administration includes an injection and a suppository. The injection includes such forms as intravenous injection, subcutaneous injection, endodermic injection, intramuscular injection, drop injection. Such injections may be prepared by dissolving, suspending or emulsifying the antibody or its salt as described above in sterile aqueous or oily liquid generally used for an injection in accordance with a publicly known method. Examples of an aqueous liquid for injection include a physiological saline and an isotonic solution containing glucose and other auxiliary agents, which may be used in combination with an appropriate dissolution aid such as an alcohol (e.g. ethanol), polyalcohol (e.g. propylene glycol, polyethylene glycol), nonionic surfactant (e.g. polysorbate 80^{TM}, HCO-50 (polyoxyethylene (50 mol) adduct of hydrogenated castor oil)). Examples of the oily liquid include sesame oil and soybean oil, which may be used in combination with a dissolution aid such as benzyl benzoate and benzyl alcohol. The injection prepared is usually filled in an appropriate ampoule. The suppository used for administration into rectum is prepared by compounding the antibody or its salt as described above with a normal base material for a suppository.

It is advantageous to prepare the above-described pharmaceutical composition for oral or parenteral use in a unit dosage form containing a suitable dose of the active component. The unit dosage form is exemplified by a table, pill, capsule, injection (ampoule), suppository. It is preferable that the unit dosage form normally contains 5 to 500 mg: particularly 5 to 100 mg for the injection and 10 to 250 mg for other forms.

These compositions as described above may contain other active components unless there generates any undesirable interaction thereof with the antibody in the mixture.

### [6] DNA-transfected animals

The present invention provides a non-human mammal having the foreign DNA encoding the protein of the present invention (abbreviated hereinafter as "the foreign DNA of the invention") or a mutated DNA thereof (sometimes abbreviated hereinafter as "the foreign mutated DNA of the present invention").

Thus, the invention provides:
(1) A non-human mammal having the foreign DNA of the present invention or the mutated DNA thereof;
(2) The animal described in (1) above, wherein the non-human mammal is a rodent;
(3) The animal described in (2) above, wherein the rodent is a mouse or rat; and
(4) A recombinant vector comprising the foreign DNA of the present invention or the mutated DNA thereof, and having the ability of expressing the DNA in a mammal.

The non-human mammal having the foreign DNA of the present invention or the mutated DNA thereof (hereinafter abbreviated as "the DNA-transfected animal of the present invention") can be prepared by transfecting the desired foreign DNA of the present invention by a method such as the calcium phosphate method, electrical pulse method, lipofection method, agglutination method, microinjection method, particle gun method or DEAE-dextran method into germ cells and the like including unfertilized eggs, fertilized eggs, sperm and primordial cells thereof, preferably during the embryonic stage of non-human mammalian development (and more preferably during the single-cell or fertilized egg cell stage, generally before the eight-cell stage). Such DNA-transfection methods can also be used to transfect the desired foreign DNA of the present invention into somatic cells, living organs or tissue cells for cell culture or tissue culture. The DNA-transfected animal of the present invention can also be produced by fusing these cells with the aforementioned germ cells according to a well-known cell fusion method.

Non-human mammals that can be used include cows, pigs, sheep, goats, rabbits, dogs, cats, guinea pigs, hamsters mice and rats. Among them, from the standpoint of preparing a pathological animal model, a rodent is preferred which has relatively short ontogeny and life cycles and which are easy to breed, especially a mouse (e.g. pure strains such as C57BL/6 and DBA2; and hybrid strains such as B6C3F₁, BDF₁, B6D2F₁, BALB/c and ICR strains) and a rat (such as Wistar and SD strain).

In the context of the recombinant vector which can express the DNA in a mammal, the term "mammal" includes a human as well as a non-human mammal.

The foreign DNA of the present invention refers to the DNA of the present invention that has been previously isolated and extracted from mammals, but not the DNA of the present invention which the non-human mammals have intrinsically.

The mutated DNA of the present invention includes a DNA having a mutation (such as various mutations) in the original nucleotide sequence of the DNA of the present invention, specifically, a DNA having addition, deletion, or substitution of a nucleic acid, and also an abnormal DNA.

The abnormal DNA refers to a DNA which expresses the abnormal protein of the present invention, and includes a DNA which expresses a protein which can inhibit the function of the normal protein of the present invention.

The foreign DNA of the present invention may be derived from a mammal of either the same species or different species from the target animal. When transfecting the DNA of the present invention into the target animal, it is generally advantageous to use a DNA construct having the DNA ligated downstream of a promoter which can function in the animal cell. For example, when transfecting the human DNA of the present invention, a DNA-transfected mammal can be prepared, which highly expresses the DNA of the present invention, by microinjecting into the fertilized eggs of the target mammal, such as fertilized mouse eggs, a DNA construct (such as a vector) having the human DNA of the present invention ligated downstream of various promoters which can express a DNA derived from various mammals (such as rabbits, dogs, cats, guinea pigs, hamsters, rats or mice) having the DNA of the present invention, which is highly homologous to the human DNA.

Plasmids derived from *E. coli, B. subtilis* or yeast, bacteriophages such as λ-phage, retroviruses such as Moloney leukemia virus and animal viruses such as vaccinia virus and baculovirus may be used as the expression vector of the protein of the present invention. Among them, plasmids derived from E. *coli, B. subtilis* or yeast are preferred.

Promoters that can be used to regulate the DNA expression include (i) promoters derived from viruses (such as simian virus, cytomegalovirus, Moloney leukemia virus, JC virus, mammary tumor virus or polio virus), and (ii) promoters derived from various mammals (humans, rabbits, dogs, cats, guinea pigs, hamsters, rats, mice, etc.), such as promoters of albumin, insulin II, uroplakin II, elastase, erythropoietin, endothelin, muscle creatine kinase, glial fibrillary acidic protein, glutathione S-transferase, platelet derived growth factor β, keratin K1, K10 and K14, collagen Type I and Type II, cyclic AMP-dependent protein kinase βI subunit, dystrophin, tartaric acid-resistant alkali phosphatase, cardiac sodium diuretic factor, endothelial receptor tyrosine kinase (normally abbreviated as Tie2), sodium-potassium ATPase (Na,K-ATPase), neurofilament light chain, metallothionein I and IIA, tissue inhibitor of metalloproteinase-1, MHC class I antigen (H-2L), H-ras, renin, dopamine β-hydroxylase, thyroid peroxidase (TPO), polypeptide chain elongation factor 1α (EF-1α), β-actin, α- and β-myosin heavy chains, myosin light chains 1 and 2, myelin basic protein, thyroglobulin, Thy-1, immunoglobulin, H chain variable region (VNP), serum amyloid P component, myoglobin, troponin C, smooth muscle α-actin, preproenkephalin A, vasopressin. Particularly suitable are cytomegalovirus promoter, human polypeptide chain elongation factor 1α (EF-1α) promoter and human and chicken β-actin promoters, which allow strong expression throughout the body.

The said vectors should preferably have the sequence (generally called the terminator) which terminates transcription of the target mRNA in DNA-transfected mammals. Terminator DNA sequences derived from viruses and mammals can be used, and the simian virus SV40 terminator is preferably used.

In order to achieve greater expression of the desired foreign DNA, it is also possible depending on the purpose to attach various DNA splicing signals, enhancer regions or parts of eukaryote-derived DNA introns at 5'-upstream of the promoter region, between the promoter region and the translation region, or at 3'-downstream of the translation region.

The translation region of the normal protein of the present invention may be obtained as a whole or part of genomic DNA from DNAs derived from liver, kidney, thyroid cells or fibroblasts of a human or various mammals (e.g. rabbits, dogs, cats, guinea pigs, hamsters, rats, mice) or from various commercial genomic DNA libraries, or may be obtained from complement DNAs prepared by a well-known method from RNAs derived from liver, kidney, thyroid cells, or fibroblasts. To prepare the abnormal foreign DNA, the translation region of normal protein obtained from the aforementioned cells or tissues can be mutated by point mutagenesis.

A DNA construct enabling the expression of the translation region in the DNA-transfected animal can be produced by a conventional genetic engineering method of inserting the translation region after the aforementioned promoter, or if desired, before the transcription termination site.

Transfection of the foreign DNA of the present invention at the fertilized egg cell stage ensures that the DNA of the present invention will be present in all germ and somatic cells of the target mammal. The presence of the foreign DNA of the present invention in the animal's germ cells after the DNA transfection means that all the animal's progenies will retain the foreign DNA of the present invention in all their germ and somatic cells. The progenies of this animal that inherit the foreign DNA of the present invention have the DNA in all their germ and somatic cells.

The non-human mammal into which the normal foreign DNA of the present invention has been transfected can be bred after confirmation of stable retention of the foreign DNA, and can be successively reared in a normal environment as an animal retaining the DNA.

Transfection of the foreign DNA of the present invention at the fertilized egg cell stage ensures that the DNA of the present invention will be present in excess in all germ and somatic cells of the target mammal. The excessive presence of the foreign DNA of the present invention in the animal's germ cells after the DNA transfection means that all the animal's progenies will retain an excess of the foreign DNA of the present invention in all their germ and somatic cells. The progenies of this animal that inherit the foreign DNA of the present invention have an excess of the foreign DNA of the present invention in all their germ and somatic cells.

It is possible to obtain homozygotic animals having the transfected DNA in both homologous chromosomes, and to breed the male and female so that all the progenies have the DNA in excess.

The normal DNA of the present invention is highly expressed in the non-human mammal having the normal DNA of the present invention, leading to the promotion of the function of the intrinsic normal DNA and ultimately to the hyperfunction of the protein of the present invention. Such an animal is useful as a pathological animal model. For example, the normal DNA-transfected animal can be used to elucidate the pathology of hyperfunction of the protein of the present invention and other diseases related to the protein of the present invention, and to investigate therapies for these conditions.

Furthermore, since the mammal into which the normal foreign DNA of the present invention is transfected has symptoms due to increased free protein of the present invention, it can also be used in screening tests for pharmaceuticals for treatment of conditions related to the protein of the present invention.

The non-human mammal having the abnormal foreign DNA of the present invention can be bred after confirmation of stable retention of the foreign DNA, and can be successively reared in a normal environment as an animal retaining the DNA. Furthermore, the desired foreign DNA can be incorporated into one of the aforementioned plasmids and used as a material. A DNA construct with a promoter can be produced according to ordinary DNA engineering techniques. Transfection of the abnormal DNA of the present invention at the fertilized egg stage ensures that the abnormal DNA of the present invention is present in all the germ and somatic cells of the target mammal. The presence of the abnormal DNA of the present invention in the animal's germ cells after the DNA transfection means that all the animal's progenies will retain the abnormal DNA of the present invention in all their germ and somatic cells. The progenies of this animal that inherit the foreign DNA will have the abnormal DNA of the present invention in all their germ and somatic cells. It is possible to obtain homozygote animals having the transfected DNA in both homologous chromosomes, and to breed the male and female so that all the progenies have this DNA.

The abnormal DNA of the present invention is highly expressed in the non-human mammal having the abnormal DNA of the present invention, leading to the inhibition of the function of the intrinsic normal DNA, and ultimately to the dysfunction of the protein of the present invention. Such an animal is useful as a pathological animal model. For example, the abnormal DNA-transfected animal can be used to elucidate the pathology of dysfunction of the protein of the present invention, and to investigate therapies for this condition.

In a specific possible application, the animal that highly expresses the abnormal DNA of the present invention could be a model for elucidating the inhibitory mechanism of normal protein function (dominant negative effect) mediated by the abnormal protein in the dysfunction of the protein of the present invention.

Moreover, since the mammal into which the abnormal foreign DNA of the present invention is transfected has symptoms due to increased free protein of the present invention, it can also be used in screening tests for pharmaceuticals for treatment of dysfunction of the protein of the present invention.

Other possible applications of the said two types of DNA-transfected animals of the present invention include:
(1) use as cell sources for tissue culture;
(2) direct analysis of DNA or RNA in the tissue of DNA-transfected mammals of the present invention or analysis of proteins expressed in tissues to elucidate the involvement of proteins that are specifically expressed or activated by the protein of the present invention;
(3) researching the function of cells derived from a tissue which is generally difficult to culture, by using cells derived from a tissue having the DNA of the present invention, wherein such cells can be cultured by standard tissue culture techniques;
(4) screening for pharmaceuticals that enhance the cellular functions using the cells described in (3) above; and
(5) isolation and purification of the mutated protein of the present invention, and production of antibodies thereto.

The DNA-transfected animals of the present invention could also be used to investigate the clinical symptoms of diseases related to the protein of the present invention, including dysfunction of the protein of the present invention, to obtain more detailed pathologies of various organs of the disease models related to the protein of the present invention, to develop new therapies, and to contribute to research and therapies for secondary conditions stemming from such diseases.

It is also possible to remove various organs from the DNA-transfected animals of the present invention, mince them, treat them with a protease such as trypsin to obtain free DNA-transfected cells, and culture the cells to prepare a cell line from the cultured cells. Since it is possible to specify the cells producing the protein of the present invention, and investigate the cells for apoptosis, differentiation and proliferation, and signal transduction, the cells can be effective research materials for understanding the protein of the present invention and action thereof.

Moreover, the DNA-transfected animals of the present invention may also be used to provide a rapid method of screening for a pharmaceutical for the treatment of diseases related to the protein of the present invention, including dysfunction of the protein of the present invention in the drug development using the assay methods and the quantifying method as described above. The DNA-transfected animals of the present invention or the vectors expressing the foreign DNA of the present invention may also be used to investigate and develop DNA therapies for diseases related to the protein of the present invention.

### [7] Knockout animals

The present invention provides a non-human mammal embryonic stem cell bearing the DNA of the invention inactivated and a non-human mammal deficient in expressing the DNA of the invention.

Thus, the present invention provides:
(1) a non-human mammal embryonic stem cell in which the DNA of the invention is inactivated;
(2) the embryonic stem cell according to (1), in which the DNA is inactivated by introducing a reporter gene (e.g. β-galactosidase gene derived from Escherichia coli);
(3) the embryonic stem cell according to (1), which is resistant to neomycin;
(4) the embryonic stem cell according to (1), wherein the non-human mammal is a rodent;
(5) the embryonic stem cell according to (4), wherein the rodent is mouse;
(6) a non-human mammal deficient in expressing the DNA of the invention, in which the DNA of the invention is inactivated;
(7) the non-human mammal according to (6), in which the DNA is inactivated by introducing a reporter gene (e.g. β-galactosidase derived from Escherichia coli) therein and the reporter gene can be expressed under the control of a promoter for the DNA of the invention;
(8) the non-human mammal according to (6), which is a rodent;
(9) the non-human mammal according to (8), wherein the rodent is a mouse; and,
(10) a method of screening for a compound that enhances or inhibits the promoter activity for the DNA of the invention, which comprises administering a test compound to the mammal of (7) and detecting expression of the reporter gene.

The non-human mammal embryonic stem cell in which the DNA of the invention is inactivated refers to the embryonic stem cells (abbreviated hereinafter as "ES cells") of a non-human mammal either in which the DNA expression ability is suppressed by the artificial mutation of the DNA of the invention present in the non-human mammal, or in which the activity of the protein of the invention encoded by said DNA has substantially been eliminated so that the DNA is not substantially capable of expressing the protein of the invention (sometimes referred to hereinafter as the knockout DNA of the invention).

The non-human mammals used are similar to those as described above.

Techniques for artificially mutating the DNA of the invention include deletion of a part or all of the DNA sequence and insertion of or substitution with other DNA, by genetic engineering. The knockout DNA of the invention may be prepared by these mutations, for example, by shifting the reading frame of a codon or by disrupting the function of a promoter or exon.

Specifically, the non-human mammal embryonic stem cell in which the DNA of the invention is inactivated (abbreviated hereinafter as the ES cells with the DNA of the invention inactivated or the knocked-out ES cells of the invention) can be produced as follows. For example, the DNA of the invention that the target non-human mammal has is isolated, a drug-resistant gene, of which typical examples are neomycin-resistant, hygromycin-resistant or other drug-resistant genes, or a reporter gene or the like, of which typical examples are lacZ (β-galactosidase gene) or cat (chloramphenicol acetyltransferase gene), is inserted into the exon to disrupt the function of the exon, or else a DNA sequence (such as polyA addition signal) which terminates the gene transcription is inserted into the intron between the exons to prevent synthesis of the complete mRNA. A DNA strand having the thus constructed DNA sequence to disrupt the gene (abbreviated hereinafter as "the targeting vector") is introduced into the chromosomes of the animal by homologous recombination. The knocked-out ES cell of the invention can be selected by analyzing the thus obtained ES cells either by the southern hybridization analysis using a DNA sequence on or near the DNA of the invention as a probe, or by the PCR analysis using as primers a DNA sequence on the targeting vector and a DNA sequence of a nearby region of the DNA of the invention used in producing the targeting vector.

The parent ES cells to inactivate the DNA of the invention by homologous recombination, etc. may be of a strain already established as described above, or may be originally established in accordance with a modification of the publicly known method by Evans and Kaufman. For example, in the case of mouse ES cells, currently it is common practice to use ES cells of the 129 strain, but their immunological genetic background is obscure. Accordingly, to establish another pure ES cell line, of which the immunological genetic background is clear, the C57BL/6 mouse or the BDF1 mouse (F1 hybrid between C57BL/6 and DBA/2), in which the low egg availability in the C57BL/6 mouse has been improved by crossing with DBA/2, may be preferably used. The BDF1 mouse is advantageous in that, when a pathologic model mouse is generated using the ES cells obtained therefrom, the genetic background can be changed to that of the C57BL/6 mouse by back-crossing with the C57BL/6 mouse, since its background is of the C57BL/6 mouse, as well as being advantageous in that egg availability is high and eggs are robust.

In establishing ES cells, blastocytes at 3.5 days after fertilization are normally used. As well, embryos can be collected at the 8-cell stage, and cultured until the blastocyte stage to efficiently obtain a large number of early stage embryos.

Although the ES cells used may be of either sex, male ES cells are generally more convenient for generation of a germ chimera cell. It is also desirable that sex of the ES cells is determined as soon as possible to save painstaking culture time.

Methods for sex determination of the ES cell include the method in which a gene in the sex-determining region on the Y-chromosome is amplified by the PCR process and detected. When this method is used, one colony of ES. cells (about 50 cells) is sufficient for sex-determination, while karyotype analysis requires about 10⁶ cells. Therefore, the first selection of ES cells at the early stage of culture can be based on sex determination, and male cells can be selected early, which saves a significant amount of time at the early stage of culture.

Second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical procedures, etc. in the cell establishment, it is desirable that the ES cell is again cloned to a normal cell (e.g. a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and 90% air) in the presence of LIF (1 to 10000 U/ml) on appropriate feeder cells such as STO fibroblasts, and treated with a trypsin/EDTA solution (normally about 0.001 to about 0.5% trypsin/about 0.1 to about 5 mM EDTA, preferably about 0.1% trypsin/1 mM EDTA) at the time of passage to obtain separate single cells, which are then seeded on freshly prepared feeder cells. The passage is normally conducted every 1 to 3 days; it is desirable that cells be observed at passage and cells found to be morphologically abnormal in culture, if any, be discarded.

By culturing ES cells to reach a high density in mono-layers or to form cell aggregates in suspension under appropriate conditions, they can differentiate to various cell types, for example, pariental and visceral muscles, cardiac muscle or the like (M. J. Evans and M. H. Kaufman, Nature, 292, 154, 1981; G. R. Martin, Proc. Natl. Acad. Sci. U.S.A., 78, 7634, 198 1; T. C. Doetschman et al., Journal of Embryology Experimental Morphology, 87, 27, 1985). The cells deficient in expression of the DNA of the invention, which are obtained from the differentiated ES cells of the invention, are useful for an in vitro cell biological study of the function of the protein of the invention.

The non-human mammal deficient in expression of the DNA of the invention can be distinguished from a normal animal by measuring the mRNA amount in the subject animal by a publicly known method, and indirectly comparing the levels of expression.

The non-human mammals used are similar to those as described above.

With respect to the non-human mammal deficient in expression of the DNA of the invention, the DNA of the invention can be knocked out by transfecting a targeting vector, prepared as described above, into mouse embryonic stem cells or egg cells thereof, and conducting homologous recombination in which the DNA sequence in the transfected targeting vector, wherein the DNA of the invention is inactivated, replaces the DNA of the invention on a chromosome of mouse embryonic stem cells or egg cells thereof.

The cells in which the DNA of the invention is knocked out can be identified either by the southern hybridization analysis using a DNA sequence on or near the DNA of the invention as a probe, or by the PCR analysis using as primers a DNA sequence on the targeting vector and another DNA sequence of a nearby region of the mouse-derived DNA of the invention used in creating the targeting vector. When using non-human mammal embryonic stem cells, a cell line in which the DNA of the invention is inactivated by homologous recombination can be cloned, and the cloned cells are injected into an embryo or blastocyst of a non-human mammal at an appropriate stage such as the 8-cell stage. The resulting chimera embryo is then transplanted to the uterus of the pseudopregnant non-human mammal. The resulting animal is a chimera animal comprising both cells having the normal locus of the DNA of the invention and the artificially mutated locus of the DNA of the invention.

When some germ cells of the chimera animal have a mutation on the locus of the DNA of the invention, an individual, whose entire tissue is composed of cells having a mutation on the locus of the DNA of the invention can be selected from a series of offsprings obtained by crossing such a chimera animal and a normal animal, e.g., by coat color identification, etc. The individuals thus obtained are normally deficient in heterozygous expression of the protein of the invention. The individuals are deficient in homozygous expression of the protein of the invention and can be obtained from offsprings of the intercross of the individuals deficient in heterozygous expression of the protein of the invention.

When using egg cells, it is possible to obtain a transgenic non-human mammal having the targeting vector inserted into the chromosomes by microinjection of the DNA solution into an egg cell nucleus. From such transgenic non-human mammals, selected is one having the mutation on the DNA locus of the invention due to homologous recombination.

The animal in which the DNA of the invention has been knocked out in this way can be successively reared in a normal environment after confirmation that the DNA is knocked out in its offsprings obtained by breeding.

Reproductive lineages can also be obtained and maintained by ordinary methods. Thus, female and male animals having the inactivated DNA can be bred to obtain homozygote animals having the inactivated DNA in both loci of homologous chromosomes. The resulting homozygote animals can be efficiently reproduced by rearing under the condition of one normal individual and multiple homozygote individuals to a mother animal. By crossing male and female heterozygotes, homozygotes and heterozygotes having the inactivated DNA are successively reproduced.

The non-human mammal embryonic stem cell, in which the DNA of the invention is inactivated, is very useful for preparing a non-human mammal deficient in expression of the DNA of the invention.

Since the non-human mammal, in which the DNA of the invention is inactivated, lacks various biological activities derived from the protein of the invention, such an animal can be a model for a disease resulted from inactivated biological activities of the protein of the invention and thus offers an effective tool to investigate the causes for and therapy for these diseases.

In the specification and drawings, bases, amino acids, and the like are abbreviated in accordance with the IUPAC-IUB Commission on Biochemical Nomenclature or the conventional usage in the art, as shown below for example. When an amino acid has optical isomers, its L form is selected unless otherwise indicated.

| | |
|---|---|
| DNA | deoxyribonucleic acid |
| cDNA | complementary deoxyribonucleic acid |
| A | adenine |
| T | thymine |
| G | guanine |
| C | cytosine |
| RNA | ribonucleic acid |
| mRNA | messenger ribonucleic acid |
| dATP | deoxyadenosine triphosphate |
| dTTP | deoxythymidine triphosphate |
| dGTP | deoxyguanosine triphosphate |
| dCTP | deoxycytidine triphosphate |
| ATP | adenosine triphosphate |
| EDTA | ethylenediaminetetraacetic acid |
| SDS | sodium dodecyl sulfate |
| Gly | glycine |
| Ala | alanine |
| Val | valine |
| Leu | leucine |
| Ile | isoleucine |
| Ser | serine |
| Thr | threonine |
| Cys | cysteine |
| Met | methionine |
| Glu | glutamic acid |
| Asp | aspartic acid |
| Lys | lysine |
| Arg | arginine |
| His | histidine |
| Phe | phenylalanine |
| Tyr | tyrosine |
| Trp | tryptophan |
| Pro | proline |
| Asn | asparagine |
| Gln | glutamine |
| pGlu | pyroglutamic acid |

Substituents, protecting groups and reagents used often in this specification are shown by the following codes.

| | |
|---|---|
| Me | methyl |
| Et | ethyl |
| Bu | butyl |
| Ph | phenyl |
| TC | thiazolidine-4(R)-carboxamido |
| Tos | p-toluenesulfonyl |
| CHO | formyl |
| Bzl | benzyl |
| Cl₂-Bzl | 2,6-dichlorobenzyl |
| Born | benzyloxymethyl |
| Z | benzyloxycarbonyl |
| Cl-Z | 2-chlorobenzyloxycarbonyl |
| Br-Z | 2-bromobenzyl oxycarbonyl |
| Boc | t-butoxycarbonyl |
| DNP | dinitrophenol |
| Trt | trityl |
| Bum | t-butoxymethyl |
| Fmoc | N-9-fluorenyl methoxycarbonyl |
| HOBt | 1-hydroxybenztriazole |
| HOOBt | 3,4-dihydro-3-hydroxy-4-oxo-1,2,3-benzotriazine |
| HONB | 1-hydroxy-5-norbornene-2,3-dicarboxyimide |
| DCC | N,N'-dichlorohexylcarbodiimide |

SEQ ID NOs in the sequence listing of the specification indicate the following sequences, respectively.

### [SEQ ID NO: 1]

This shows the amino acid sequence of a novel rat-derived protein of the invention (rat MEF-2C, i.e. rMEF2ChW) obtained in Example 1.

### [SEQ ID NO: 2]

This shows the amino acid sequence of a novel rat-derived protein of the invention (rMEF2ChV1) obtained in Example 1.

### [SEQ ID NO: 3]

This shows the amino acid sequence of a novel rat-derived protein of the invention (rMEF2ChV2) obtained in Example 1.

### [SEQ ID NO: 4]

This shows the base sequence of the DNA encoding the protein having the amino acid sequence represented by SEQ ID NO: 1.

### [SEQ ID NO: 5]

This shows the base sequence of the DNA encoding the protein having the amino acid sequence represented by SEQ ID NO: 2.

### [SEQ ID NO: 6]

This shows the base sequence of the DNA encoding the protein having the amino acid sequence represented by SEQ ID NO: 3.

### [SEQ ID NO: 7]

This shows a partial sequence of the base sequence represented by SEQ ID NO: 4.

### [SEQ ID NO: 8]

This shows the base sequence of AP1 primer used in Example 1.

### [SEQ ID NO: 9]

This shows the base sequence of a primer used in Example 1.

### [SEQ ID NO: 10]

This shows the base sequence of a primer used in Example 1.

### [SEQ ID NO: 11]

This shows the base sequence of a primer used in Example 1.

### [SEQ ID NO: 12]

This shows the base sequence of a primer used in Example 1.

### [SEQ ID NO: 13]

This shows the base sequence of T7 primer used in Example 1.

### [SEQ ID NO: 14]

This shows the base sequence of SP6 primer used in Example 1.

### [SEQ ID NO: 15]

This shows the amino acid sequence of a novel rat-derived protein of the invention (rMEF2CbW) obtained in Example 2.

### [SEQ ID NO: 16]

This shows the amino acid sequence of a novel rat-derived protein of the invention (rMEF2CbV1) obtained in Example 2.

### [SEQ ID NO: 17]

This shows the amino acid sequence of a novel rat-derived protein of the invention (rMEF2CbV2) obtained in Example 2.

### [SEQ ID NO: 18]

This shows the base sequence of the DNA encoding the protein having the amino acid sequence represented by SEQ ID NO: 15.

### [SEQ ID NO: 19]

This shows the base sequence of the DNA encoding the protein having the amino acid sequence represented by SEQ ID NO: 16.

### [SEQ ID NO: 20]

This shows the base sequence of the DNA encoding the protein having the amino acid sequence represented by SEQ ID NO: 17.

### [SEQ ID NO: 21]

This shows the amino acid sequence of human MEF2C protein.

### [SEQ ID NO: 22]

This shows the amino acid sequence of mouse MEF2C protein.

### [SEQ ID NO: 23]

This shows the amino acid sequence of a splicing variant of mouse MEF2C protein.

The transformant Escherichia coli TOP10F'/pTB2207, obtained in Example 1 is deposited at Institute for Fermentation, Osaka (IFO) located at 2-17-85, Jyuso-Honmati, Yodogawa-ku, Osaka-shi, Osaka 532-8686, Japan, under Accession Number IFO 16532 since February 1, 2001; and at International Patent Organism Depositary, National Institute of Advanced Industrial Science and Technology (now-defunct National Institute of Bioscience and Human Technology (NIBH), Agency of Industrial Science and Technology, Ministry of International Trade and Industry) located at Center No. 6, 1-1-1 Higasi, Tukuba-shi, Ibaraki 305-8566, Japan, under Accession Number FERM BP-7465 since February 22,2001.

The transformant Escherichia coli TOP10F'/pTB2208, obtained in Example 1 is deposited at Institute for Fermentation, Osaka (IFO) located at 2-17-85, Jyuso-Honmati, Yodogawa-ku, Osaka-shi, Osaka 532-8686, Japan, under Accession Number IFO 16533 since February 1,2001; and at International Patent Organism Depositary, National Institute of Advanced Industrial Science and Technology (now-defunct National Institute of Bioscience and Human Technology (NIBH), Agency of Industrial Science and Technology, Ministry of International Trade and Industry) located at CenterNo. 6, 1-1-1 Higasi, Tukuba-shi, Ibaraki 305-8566, Japan, under Accession Number FERM BP-7466 since February 22, 2001.

The transformant Escherichia coli TOP10F'/pTB2209, obtained in Example 1 is deposited at Institute for Fermentation, Osaka (IFO) located at 2-17-85, Jyuso-Honmati, Yodogawa-ku, Osaka-shi, Osaka 532-8686, Japan, under Accession Number IFO 16534 since February 1,2001; and at International Patent Organism Depositary, National Institute of Advanced Industrial Science and Technology (now-defunct National Institute of Bioscience and Human Technology (NIBH), Agency of Industrial Science and Technology, Ministry of International Trade and Industry) located at Center No. 6, 1-1-1 Higasi, Tukuba-shi, Ibaraki 305-8566, Japan, under Accession Number FERM BP-7467 since February 22, 2001.

The transformant Escherichia coli DH5α/pTB2234, obtained in Example 2 is deposited at Institute for Fermentation, Osaka (IFO) located at 2-17-85, Jyuso-Honmati, Yodogawa-ku, Osaka-shi, Osaka 532-8686, Japan, under Accession Number IFO 16759 since February 14, 2002; and at International Patent Organism Depositary, National Institute of Advanced Industrial Science and Technology located at Center No. 6, 1-1-1 Higasi, Tukuba-shi, Ibaraki 305-8566, Japan, under Accession Number FERM BP-7928 since February 27, 2002.

The transformant Escherichia coli DH5α/pTB2235, obtained in Example 2 is deposited at Institute for Fermentation, Osaka (IFO) located at 2-17-85, Jyuso-Honmati, Yodogawa-ku, Osaka-shi, Osaka 532-8686, Japan, under Accession Number IFO 16760 since February 14, 2002; and at International Patent Organism Depositary, National Institute of Advanced Industrial Science and Technology located at Center No. 6, 1-1-1 Higasi, Tukuba-shi, Ibaraki 305-8566, Japan, under Accession Number FERM BP-7929 since February 27, 2002.

The transformant Escherichia coli DH5α/pTB2236, obtained in Example 2 is deposited at Institute for Fermentation, Osaka (IFO) located at 2-17-85, Jyuso-Honmati, Yodogawa-ku, Osaka-shi, Osaka 532-8686, Japan, under Accession Number IFO 16761 since February 14, 2002; and at International Patent Organism Depositary, National Institute of Advanced Industrial Science and Technology located at Center No. 6, 1-1-1 Higasi, Tukuba-shi, Ibaraki 305-8566, Japan, under Accession Number FERM BP-7930 since February 27, 2002.

### EXAMPLES

The invention is specifically described below with reference to Examples, but not limited thereto. Genetic procedures using Escherichia coli followed methods described in Molecular Cloning, 2nd Ed. (J. Sambrook et al., Cold Spring Harbor Lab. Press, 1989).

### EXAMPLE 1

Based on SEQ ID NO: 7 (GenBank Accession No. AA955670; a partial sequence of rat MEF-2C gene), the full-length of the gene fragment was cloned by 5' RACE and 3' RACE.

Briefly, a PCR was performed using Marathon-Ready heart cDNA library (Clontech) as a template and two primer DNAs: AP1 primer (SEQ ID NO: 8) and a primer having the nucleotide sequence as shown in SEQ ID NO: 9 to thereby clone the 5' upstream region. Further, a PCR was performed using two primer DNAs: AP1 primer (SEQ ID NO: 8) and a primer having the nucleotide sequence as shown in SEQ ID NO: 10 to thereby clone the 3' upstream region. Based on these sequences, a PCR was performed using two primer DNAs: 5' upstream primer (SEQ ID NO: 11) and 3' downstream primer (SEQ ID NO: 12) to thereby obtain three ORFs (SEQ ID NOS: 4, 5 and 6). The PCR reactions were performed using Pfu DNA polymerase (Toyobo) on a thermal cycler gene amp PCR system 9700 (Perkin-Elmer) through 35 cycles, each cycle consisting of 97°C for 10 sec and 68°C for 4 min.

DNAs having the resultant nucleotide sequences as shown in SEQ ID NOS: 4, 5 and 6, respectively, were cloned into pCRII-TOPO vector using TOPO TA Cloning Kit (Invitrogen). The resultant plasmids were designated pTB2207, pTB2208 and pTB2209, respectively. Further, a reaction was performed using known synthetic primers [T7 primer (SEQ ID NO: 13) and SP6 primer (SEQ ID NO: 14)] and Cycle Sequence Kit (PE Applied Biosystems). The nucleotide sequence of the resultant product was determined by a fluorescent DNA sequencer (ABI PRISM 377; Perkin-Elmer). As a result, it was confirmed that the above DNAs are genes encoding novel MEF-2C proteins.

The transformants obtained by introducing the above-mentioned plasmids pTB2207, pTB2208 and pTB2209 into *Escherichia coli* were designated *Escherichia coli* TOP1 OF'/ pTB2207, *Escherichia coli* TOP10F'/ pTB2208 and *Escherichia coli* TOP10F'/ pTB2209, respectively.

The novel protein having the amino acid sequence (SEQ ID NO: 1) encoded by the DNA sequence represented by SEQ ID NO: 4 was designated rat MEF-2C (rMEF2ChW). The novel protein having the amino acid sequence (SEQ ID NO: 2) encoded by the DNA sequence represented by SEQ ID NO: 5 was designated rMEF2ChV1. The novel protein having the amino acid sequence (SEQ ID NO: 3) encoded by the DNA sequence represented by SEQ ID NO: 6 was designated rMEF2ChV2.

### EXAMPLE 2

Based on SEQ ID NO: 4, the full-length of the gene was cloned by PCR.

Briefly, a PCR was performed using Marathon-Ready brain cDNA library (Clontech) as a template and two primer DNAs: 5' upstream primer (SEQ ID NO: 11) and 3' upstream primer (SEQ ID NO: 12) to thereby obtain four different ORFs (SEQ ID NOS: 2, 18, 19 and 20). One of them was identical with rMEF2ChV1 (SEQ ID NO: 2). The PCR reaction was performed using Pyrobest DNA polymerase (Takara Shuzo) on a thermal cycler gene amp PCR system 9700 (Perkin-Elmer) through 30 cycles, each cycle consisting of 94°C for 10 sec and 68°C for 2 min and 30 sec.

DNAs having the nucleotide sequences as shown in SEQ ID NOS: 18, 19 and 20, respectively, were cloned into pCRII-TOPO vector using TOPO TA Cloning Kit (Invitrogen). The resultant plasmids were designated pTB2234, pTB2235 and pTB2236, respectively. Further, a reaction was performed using known synthetic primers [T7 primer (SEQ ID NO: 13) and SP6 primer (SEQ ID NO: 14)] and Cycle Sequence Kit (PE Applied Biosystems). The nucleotide sequence of the resultant product was determined by a fluorescent DNA sequencer (ABI PRISM 377; Perkin-Elmer). As a result, it was confirmed that the above DNAs are genes encoding novel MEF-2C proteins.

The transformants obtained by introducing the above-mentioned plasmids pTB2234, pTB2235 and pTB2236 into *Escherichia* coli were designated *Escherichia coli* DH5α/pTB2234, *Escherichia coli* DH5α/pTB2235 and *Escherichia coli* DH5α/pTB2236, respectively.

The novel protein having the amino acid sequence (SEQ ID NO: 15) encoded by the DNA sequence represented by SEQ ID NO: 18 was designated rMEF2CbW. The novel protein having the amino acid sequence (SEQ ID NO: 16) encoded by the DNA sequence represented by SEQ ID NO: 19 was designated rMEF2CbV1. The novel protein having the amino acid sequence (SEQ ID NO: 17) encoded by the DNA sequence represented by SEQ ID NO: 20 was designated rMEF2CbV2.

### EXAMPLE 3

A heart was removed from a newborn rat, washed with phosphate buffer, and then cut into pieces. The tissue pieces were washed with the same buffer to remove blood cells and then digested with trypsin and collagenase. The digestive reaction was terminated with 10% serum-containing M199 medium (Gibco). Then, cells were harvested by centrifugation, suspended in the medium and plated onto petri dishes. After 1-hour culture at 37°C, floating cells were collected and treated with a hypotonic solution to thereby disrupt erythrocytes. After centrifugation, cells were suspended in the medium and plated in petri dishes at 3.0 x 10⁵ cells/well (12-well plates). After 24-hour culture, petri dishes were agitated gently and then the medium was exchanged. As a result, myocardial cells were obtained at high purity.

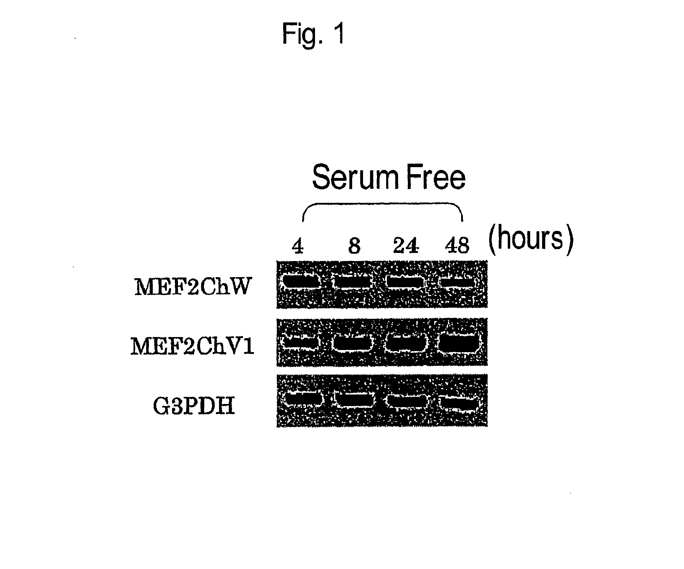

The culture medium for these myocardial cells was exchanged with serum-not-containing M199 medium (serum-free medium) to start the cultivation. The expression levels of MEF2ChW gene, MEF2ChV1 gene and G3PDH gene (as an internal standard) at 4, 8, 24 and 48 hours after the exchange with the serum-free medium were quantitatively determined by RT-PCR. The results are shown in Fig. 1.

From these results, it has become clear that the expression level of MEF2ChW gene decreases with time and that the expression level of MEF2ChV1 gene increases with time.

### EXAMPLE 4

The culture medium for the highly pure myocardial cells obtained by the method described in Example 3 is exchanged with serum-not-containing M199 medium (serum-free medium) (Gibco) to start the cultivation. After the exchange with the serum-free medium, a test compound is added to the culture and the expression levels of MEF2ChW gene and MEF2ChV1 gene at 4, 8, 24 and 48 hours after the exchange are quantitatively measured by TaqMan-PCR or RT-PCR. In the same manner, the expression levels of MEF2ChW gene and MEF2ChV1 gene in the absence of the test compound are quantitatively measured by TaqMan-PCR or RT-PCR.

The expression levels of the genes in the both cases are quantitatively compared with a densitometer. A test compound which inhibits or promotes the expression of MEF2ChW gene and MEF2ChV1 gene compared to the expression levels in the absence of the test compound is selected as a compound that inhibits or promotes the activity of MEF2ChW and MEF2ChV1.

### EXAMPLE 5

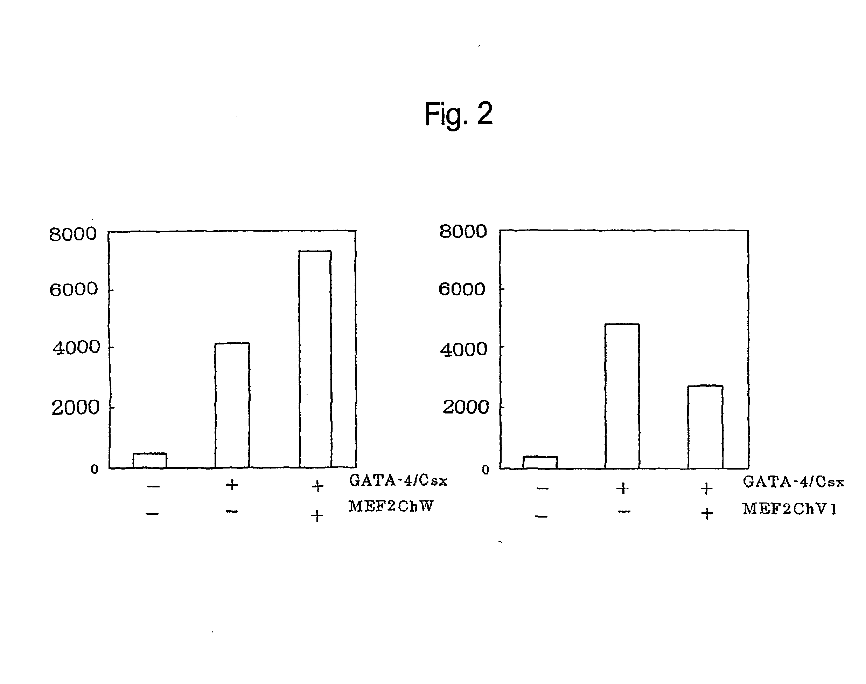

(1) pGL3-Basic plasmid (Promega) in which luciferase gene was linked downstream of the promoter of atrial natriuretic factor (ANF), (2) pCDNA3.1⁺ plasmid (Invitrogen) into which GATA-4 gene was subcloned, (3) pCDNA3.1⁺plasmid (Invitrogen) into which Nkx2.5/Csx gene was subcloned, and (4) pCDNA3.1⁺ plasmid (Invitrogen) into which MEF2ChW gene or MEF2ChV gene was subcloned, were introduced by electroporation into myocardial cells prepared according to the method described in Example 3 . The expression level of the luciferase gene 48 hours after the start of the cultivation was measured with a fluorophotometer. GATA-4 protein and Nkx2.5/Csx protein bind to ANF promoter to promote the expression of luciferase gene. The results are shown in Fig. 2. The vertical axis represents fluorescence intensity.

These results revealed that the expression of luciferase gene which is promoted by GATA-4 gene and Nkx2.5/Csx gene is increased further by MEF2ChW gene, whereas MEF2ChV 1 gene decreases that expression.

### EXAMPLE 6

(1) pGL3-Basic plasmid (Promega) in which luciferase gene is linked downstream of the promoter of atrial natriuretic factor (ANF), (2) pCDNA3.1⁺ plasmid (Invitrogen) into which GATA-4 gene is subcloned, (3) pCDNA3.1⁺ plasmid (Invitrogen) into which Nkx2.5/Csx gene is subcloned, and (4) pCDNA3.1⁺ plasmid (Invitrogen) into which MEF2ChW gene or MEF2ChV1 gene is subcloned are introduced by electroporation into highly pure myocardial cells prepared according to the method described in Example 3 or HeLa cells. At 8, 24 and 48 hours after a test compound is added to these cells, the expression level of the reporter gene is measured with a fluorophotometer. A compound that increases or decreases the fluorescence intensity is selected as a compound that inhibits or promotes the activity of MEF2ChW gene or MEF2ChV 1 gene.

### EXAMPLE 7

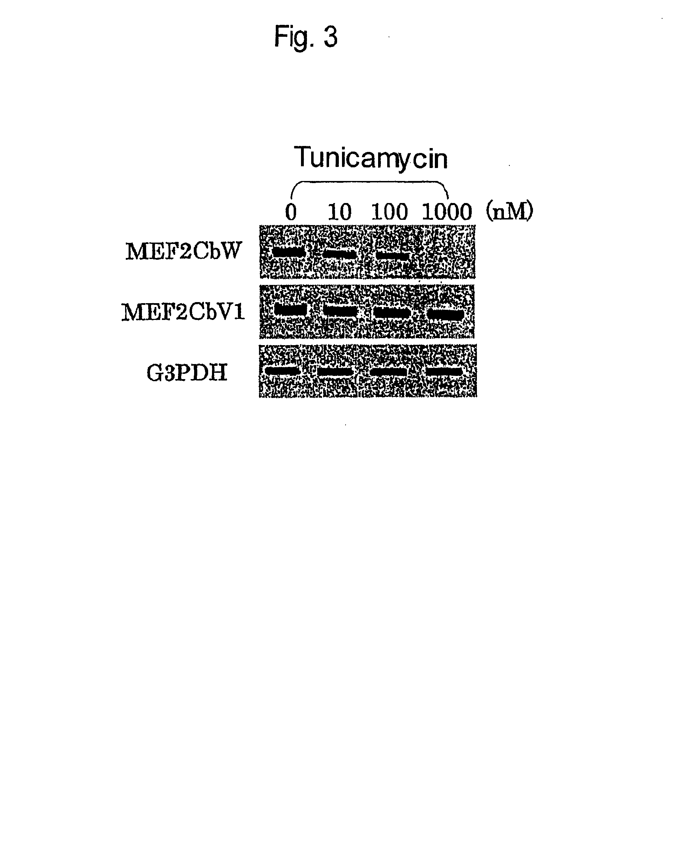

The hippocampus was removed from rat fetal brain, and hippocampal nerve cells were prepared based on the method described in the instructions attached to Nerve Cell Separation Kit (Sumitomo Bakelite). These cells were suspended in Neurobasal medium containing B27 additive (Gibco) and plated onto petri dishes at 2.5 x 10⁴ cells/well (24-well plates). After 72-hour culture at 37°C, tunicamycin was added to the cells to give stress on endoplasmic reticulum. Eight hours thereafter, the expression levels of MEF2CbW gene, MEF2CbV 1 gene and G3PDH gene (as an internal standard) were quantitatively determined by RT-PCR. The results are shown in Fig. 3.

These results revealed that the expression of MEF2CbW gene decreases remarkably with time, whereas the expression of MEF2CbV 1 gene increases gradually with time.

### EXAMPLE 8

The hippocampal nerve cells prepared in Example 7 are suspended in Neurobasal medium containing B27 additive (Gibco) and plated onto petri dishes at 2.5 x 10⁴ cells/well (24-well plates). After 72-hour culture at 37°C, a test compound and tunicamycin are added to the cells. Eight hours thereafter, the expression levels of MEF2CbW gene, MEF2CbV 1 gene and G3PDH gene (as an internal standard) are quantitatively determined by TaqMan-PCR or RT-PCR. The expression levels in the presence of a test compound and in the absence of the test compounds are quantitatively compared. Then, a test compound that inhibits or promotes the expression of MEF2CbW gene and MEF2CbV 1 gene compared to their expression in the absence of the test compound is selected as a compound that inhibits or promotes the activity of MEF2CbW gene and MEF2CbV 1 gene.

### EXAMPLE 9

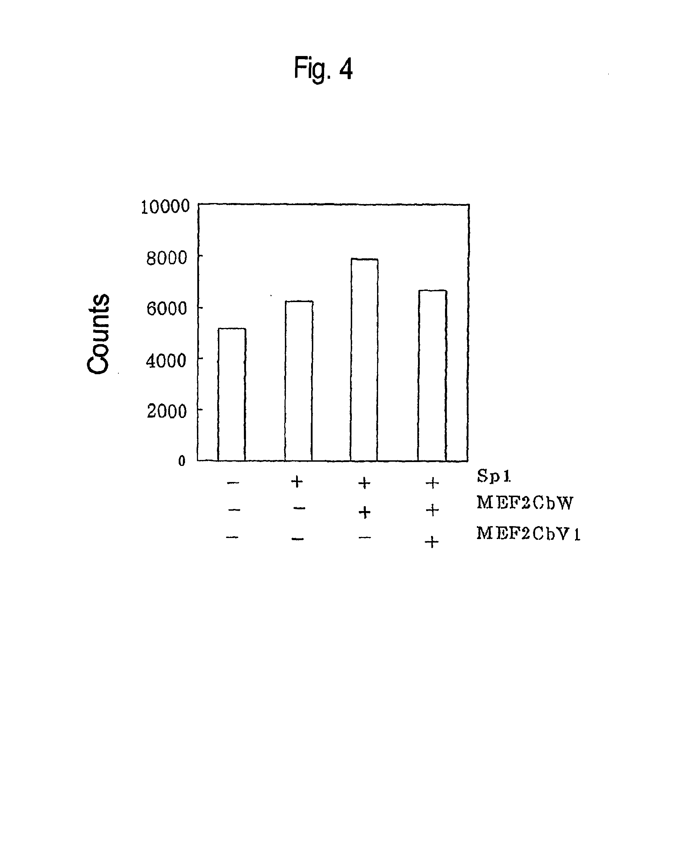

(1) A plasmid (NMDA-pGL3) in which luciferase gene was linked downstream of the promoter of N-methyl-D-aspartate receptor (NMDA), (2) pCDNA3.1 ⁺ plasmid (Invitrogen) into which Sp-1 gene was subcloned, and (3) pCDNA3.1⁺ plasmid (Invitrogen) into which MEF2CbW gene or MEF2CbV 1 gene was subcloned, were introduced into PC12 cells (ATCC: CRL-1721) by the Lipofectin method. The expression level of the reporter gene was measured 48 hours after the start of the culture. Sp-1 gene binds to NMDA promoter to promote the expression of luciferase gene. The results are shown in Fig. 4.

These results revealed that the expression of luciferase gene which is enhanced by Sp-1 gene is increased further by MEF2ChW gene, and that MEF2ChV 1 gene inhibits the increase in the luciferase gene expression caused by MEF2ChW gene.

### EXAMPLE 10

( 1) A plasmid (NMDA-pGL3) in which luciferase gene is linked downstream of the promoter of N-methyl-D-aspartate receptor (NMDA), (2) pCDNA3.1⁺ plasmid (Invitrogen) into which Sp-1 gene is subcloned, and (3) pCDNA3.1⁺ plasmid (Invitrogen) into which MEF2CbW gene or MEF2CbV1 gene is subcloned, are introduced by the Lipofectin method into PC 12 cells (ATCC: CRL-1721) or the hippocampus-derived nerve cells prepared in Example 7. At 8, 24 and 48 hours after a test compound is added to these cells, the expression level of the reporter gene is measured with a fluorophotometer. A compound which increases or decreases the fluorescence intensity is selected as a compound that inhibits or promotes the activity of MEF2CbW gene or MEF2CbV 1 gene.

### INDUSTRIAL APPLICABILITY

The protein comprising the same or substantially the same amino acid sequence as represented by SEQ ID NO: 1,2, 3,15,16 or 17 or its salt is novel. The compound or its salt regulating the activity of the protein or its salt, and the antibody regulating the activity of the protein or its salt can be used as a prophylactic and/or therapeutic agent for heart diseases, central nervous system diseases, and the like. The antisense nucleotide having a base sequence complementary or substantially complementary to the DNA encoding the protein or its salt is capable of inhibiting the expression of the protein or its salt, and thus is useful as a prophylactic and/or therapeutic agent for heart diseases, central nervous system diseases, and the like.

## Claims

1. A protein comprising the same or substantially the same amino acid sequence as the amino acid sequence shown by SEQ ID NO: 1, 2, 3, 15, 16 or 17; or a salt thereof.

2. The protein according to claim 1 or a salt thereof, which comprises the same or substantially the same amino acid sequence as the amino acid sequence shown by SEQ ID NO: 1, 2 or 3.

3. The protein according to claim 1 or a salt thereof, which comprises the amino acid sequence shown by SEQ ID NO: 1.

4. The protein according to claim 1 or a salt thereof, which comprises the amino acid sequence shown by SEQ ID NO: 2.

5. The protein according to claim 1 or a salt thereof, which comprises the amino acid sequence shown by SEQ ID NO: 3.

6. The protein according to claim 1 or a salt thereof, which comprises the amino acid sequence shown by SEQ ID NO: 15.

7. The protein according to claim 1 or a salt thereof, which comprises the amino acid sequence shown by SEQ ID NO: 16.

8. The protein according to claim 1 or a salt thereof, which comprises the amino acid sequence shown by SEQ ID NO: 17.

9. A partial peptide of the protein according to claim 1, or a salt thereof.

10. A polynucleotide comprising a polynucleotide encoding the protein according to claim 1 or the partial peptide according to claim 9.

11. The polynucleotide according to claim 10, which is DNA.

12. The polynucleotide according to claim 11, which comprises the nucleotide sequence represented by SEQ ID NO: 4, 5, 6, 18, 19 or 20.

13. A recombinant vector comprising the polynucleotide according to claim 10.

14. A transformant transformed by the recombinant vector according to claim 13.

15. A method of producing the protein according to claim 1 or a salt thereof, or the partial peptide according to claim 9 or a salt thereof, which comprises culturing the transformant according to claim 14 to produce and accumulate the protein according to claim 1 or the partial peptide according to claim 9; and collecting it.

16. An antibody to the protein according to claim 1 or a salt thereof or the partial peptide according to claim 9 or a salt thereof.

17. A pharmaceutical composition comprising the antibody according to claim 16.

18. A diagnostic composition comprising the antibody according to claim 16.

19. A method of screening for a compound or a salt thereof regulating the activity of the protein according to claim 1 or a salt thereof or the partial peptide according to claim 9 or a salt thereof, which comprises using the protein according to claim 1 or a salt thereof or the partial peptide according to claim 9 or a salt thereof.

20. A kit for screening for a compound or a salt thereof regulating the activity of the protein according to claim 1 or a salt thereof or the partial peptide according to claim 9 or a salt thereof, which comprises the protein according to claim 1 or a salt thereof or the partial peptide according to claim 9 or a salt thereof.

21. A compound or a salt thereof regulating the activity of the protein according to claim 1 or a salt thereof or the partial peptide according to claim 9 or a salt thereof, which can be obtained by the screening method according to claim 19 or the screening kit according to claim 20.

22. A pharmaceutical composition comprising the compound according to claim 21 or a salt thereof.

23. A method of screening for a compound or a salt thereof regulating the gene expression of the protein according to claim 1, which comprises using the polynucleotide according to claim 10.

24. A method of screening for a compound or a salt thereof enhancing the gene expression of the protein comprising the same or substantially the same amino acid sequence as the amino acid sequence shown by SEQ ID NO: 1 or 15, which comprises using a polynucleotide encoding the protein.

25. A method of screening for a compound or a salt thereof inhibiting the gene expression of the protein comprising the same or substantially the same amino acid sequence as the amino acid sequence shown by SEQ ID NO: 2, 3, 16 or 17, which comprises using a polynucleotide encoding the protein.

26. A kit for screening for a compound or a salt thereof regulating the gene expression of the protein according to claim 1, which comprises the polynucleotide according to claim 10.

27. A compound or a salt thereof regulating the gene expression of the protein according to claim 1, which can be obtained by the screening method according to claim 23 or the screening kit according to claim 26.

28. A compound or a salt thereof enhancing the gene expression of the protein comprising the amino acid sequence shown by SEQ ID NO: 1 or 15, which can be obtained by the screening method according to claim 24.

29. A compound or a salt thereof inhibiting the gene expression of the protein comprising the amino acid sequence shown by SEQ ID NO: 2, 3, 16 or 17, which can be obtained by the screening method according to claim 25.

30. A pharmaceutical composition comprising the compound according to claim 27, 28 or 29 or a salt thereof.

31. An antisense polynucleotide comprising a nucleotide sequence complementary to the polynucleotide according to claim 10 or a partial sequence thereof.

32. A pharmaceutical composition comprising the antisense polynucleotide according to claim 31.

33. The pharmaceutical composition according to claim 17, 22, 30 or 32, which is a prophylactic and/or therapeutic agent for heart diseases or central nervous system diseases.

34. A method of preventing and/or treating heart diseases or central nervous system diseases in a mammal, which comprises administering an effective amount of the compound according to claim 21, 27, 28 or 29 or a salt thereof to the mammal.

35. A use of the compound according to claim 21, 27, 28 or 29 or a salt thereof for producing a prophylactic and/or therapeutic agent for heart diseases or central nervous system diseases.


SEQUENCE LISTING

<110> Takeda Chemical Industries, Ltd.

<120> Novel protein, DNA thereof and use of the same

<130> P02-0046PCT

<140> PCT/JP02/02841
<141> 2002-03-25

<150> JP 2001-088041
<151> 2001-03-26

<150> JP 2001-258443
<151> 2001-08-28

<160> 23

<210> 1
<211> 465
<212> PRT
<213> Rat

<400> 1
Met Gly Arg Lys Lys Ile Gln Ile Thr Arg Ile Met Asp Glu Arg Asn
                5                   10                  15
Arg Gln Val Thr Phe Thr Lys Arg Lys Phe Gly Leu Met Lys Lys Ala
            20                  25                  30
Tyr Glu Leu Ser Val Leu Cys Asp Cys Glu Ile Ala Leu Ile Ile Phe
        35                  40                  45
Asn Ser Thr Asn Lys Leu Phe Gln Tyr Ala Ser Thr Asp Met Asp Lys
    50                  55                  60
Val Leu Leu Lys Tyr Thr Glu Tyr Asn Glu Pro His Glu Ser Arg Thr
65                  70                  75                  80
Asn Ser Asp Ile Val Glu Thr Leu Arg Lys Lys Gly Leu Asn Gly Cys

85                 90                 95
Asp Ser Pro Asp Pro Asp Ala Asp Asp Ser Val Gly His Ser Pro Glu
            100                 105                 110
Ser Glu Asp Lys Tyr Arg Lys Ile Asn Glu Asp Ile Asp Leu Met Ile
        115                 120                 125
Ser Arg Gln Arg Leu Cys Ala Val Pro Pro Pro Asn Phe Glu Met Pro
    130                 135                 140
Val Thr Ile Pro Val Ser Ser His Asn Ser Leu Val Tyr Ser Asn Pro
145                 150                 155                 160
Val Ser Ser Leu Gly Asn Pro Asn Leu Leu Pro Leu Ala His Pro Ser
                165                 170                 175
Leu Gln Arg Asn Ser Met Ser Pro Gly Val Thr His Arg Pro Pro Ser
            180                 185                 190
Ala Gly Asn Thr Gly Gly Leu Met Gly Gly Asp Leu Thr Ser Gly Ala
        195                 200                 205
Gly Thr Ser Ala Gly Asn Gly Tyr Gly Asn Pro Arg Asn Ser Pro Gly
    210                 215                 220
Leu Leu Val Ser Pro Gly Asn Leu Asn Lys Asn Ile Gln Ala Lys Ser
225                 230                 235                 240
Pro Pro Pro Met Asn Leu Gly Met Asn Asn Arg Lys Pro Asp Leu Arg
                245                 250                 255
Val Leu Ile Pro Pro Gly Ser Lys Asn Thr Met Pro Ser Val Asn Gln
            260                 265                 270
Arg Ile Asn Asn Ser Gln Ser Ala Gln Ser Leu Ala Thr Pro Val Val
        275                 280                 285
Ser Val Ala Thr Pro Thr Leu Pro Gly Gln Gly Met Gly Gly Tyr Pro
    290                 295                 300
Ser Ala Ile Ser Thr Thr Tyr Gly Thr Glu Tyr Ser Leu Ser Ser Ala
305                 310                 315                 320
Asp Leu Ser Ser Leu Ser Gly Phe Asn Thr Ala Ser Ala Leu His Leu
                325                 330                 335
Gly Ser Val Thr Gly Trp Gln Gln Gln His Leu His Asn Met Pro Pro
            340                 345                 350
Ser Ala Leu Ser Gln Leu Gly Ala Cys Thr Ser Thr His Leu Ser Gln
        355                 360                 365
Ser Ser Asn Leu Ser Leu Pro Ser Thr Gln Ser Leu Asn Ile Lys Ser

370                 375                 380
Glu Pro Val Ser Pro Pro Arg Asp Arg Thr Thr Thr Pro Ser Arg Tyr
385                 390                 395                 400
Pro Gln His Thr Arg His Glu Ala Gly Arg Ser Pro Val Asp Ser Leu
                405                 410                 415
Ser Ser Cys Ser Ser Ser Tyr Asp Gly Ser Asp Arg Glu Asp His Arg
            420                 425                 430
Asn Glu Phe His Ser Pro Ile Gly Leu Thr Arg Pro Ser Pro Asp Glu
        435                 440                 445
Arg Glu Ser Pro Ser Val Lys Arg Met Arg Leu Ser Glu Gly Trp Ala
    450                 455                 460
Thr
465

<210> 2
<211> 433
<212> PRT
<213> Rat

<400> 2
Met Gly Arg Lys Lys Ile Gln Ile Thr Arg Ile Met Asp Glu Arg Asn
                5                   10                  15
Arg Gln Val Thr Phe Thr Lys Arg Lys Phe Gly Leu Met Lys Lys Ala
            20                  25                  30
Tyr Glu Leu Ser Val Leu Cys Asp Cys Glu Ile Ala Leu Ile Ile Phe
        35                  40                  45
Asn Ser Thr Asn Lys Leu Phe Gln Tyr Ala Ser Thr Asp Met Asp Lys
    50                  55                  60
Val Leu Leu Lys Tyr Thr Glu Tyr Asn Glu Pro His Glu Ser Arg Thr
65                  70                  75                  80
Asn Ser Asp Ile Val Glu Thr Leu Arg Lys Lys Gly Leu Asn Gly Cys
                85                  90                  95
Asp Ser Pro Asp Pro Asp Ala Asp Asp Ser Val Gly His Ser Pro Glu
            100                 105                 110
Ser Glu Asp Lys Tyr Arg Lys Ile Asn Glu Asp Ile Asp Leu Met Ile
        115                 120                 125

Ser Arg Gln Arg Leu Cys Ala Val Pro Pro Pro Asn Phe Glu Met Pro
    130                 135                 140
Val Thr Ile Pro Val Ser Ser His Asn Ser Leu Val Tyr Ser Asn Pro
145                 150                 155                 160
Val Ser Ser Leu Gly Asn Pro Asn Leu Leu Pro Leu Ala His Pro Ser
                165                 170                 175
Leu Gln Arg Asn Ser Met Ser Pro Gly Val Thr His Arg Pro Pro Ser
            180                 185                 190
Ala Gly Asn Thr Gly Gly Leu Met Gly Gly Asp Leu Thr Ser Gly Ala
        195                 200                 205
Gly Thr Ser Ala Gly Asn Gly Tyr Gly Asn Pro Arg Asn Ser Pro Gly
    210                 215                 220
Leu Leu Val Ser Pro Gly Asn Leu Asn Lys Asn Ile Gln Ala Lys Ser
225                 230                 235                 240
Pro Pro Pro Met Asn Leu Gly Met Asn Asn Arg Lys Pro Asp Leu Arg
                245                 250                 255
Val Leu Ile Pro Pro Gly Ser Lys Asn Thr Met Pro Ser Val Asn Gln
            260                 265                 270
Arg Ile Asn Asn Ser Gln Ser Ala Gln Ser Leu Ala Thr Pro Val Val
        275                 280                 285
Ser Val Ala Thr Pro Thr Leu Pro Gly Gln Gly Met Gly Gly Tyr Pro
    290                 295                 300
Ser Ala Ile Ser Thr Thr Tyr Gly Thr Glu Tyr Ser Leu Ser Ser Ala
305                 310                 315                 320
Asp Leu Ser Ser Leu Ser Gly Phe Asn Thr Ala Ser Ala Leu His Leu
                325                 330                 335
Gly Ser Val Thr Gly Trp Gln Gln Gln His Leu His Asn Met Pro Pro
            340                 345                 350
Ser Ala Leu Ser Gln Leu Gly Asp Arg Thr Thr Thr Pro Ser Arg Tyr
        355                 360                 365
Pro Gln His Thr Arg His Glu Ala Gly Arg Ser Pro Val Asp Ser Leu
    370                 375                 380
Ser Ser Cys Ser Ser Ser Tyr Asp Gly Ser Asp Arg Glu Asp His Arg
385                 390                 395                 400
Asn Glu Phe His Ser Pro Ile Gly Leu Thr Arg Pro Ser Pro Asp Glu
                405                 410                 415

Arg Glu Ser Pro Ser Val Lys Arg Met Arg Leu Ser Glu Gly Trp Ala
            420                 425                 430
Thr

<210> 3
<211> 431
<212> PRT
<213> Rat

<400> 3
Met Gly Arg Lys Lys Ile Gln Ile Thr Arg Ile Met Asp Glu Arg Asn
                5                   10                  15
Arg Gln Val Thr Phe Thr Lys Arg Lys Phe Gly Leu Met Lys Lys Ala
            20                  25                  30
Tyr Glu Leu Ser Val Leu Cys Asp Cys Glu Ile Ala Leu Ile Ile Phe
        35                  40                  45
Asn Ser Thr Asn Lys Leu Phe Gln Tyr Ala Ser Thr Asp Met Asp Lys
    50                  55                  60
Val Leu Leu Lys Tyr Thr Glu Tyr Asn Glu Pro His Glu Ser Arg Thr
65                  70                  75                  80
Asn Ser Asp Ile Val Glu Ala Leu Asn Lys Lys Glu Asn Lys Gly Ser
                85                  90                  95
Glu Ser Pro Asp Pro Asp Ser Ser Tyr Ala Leu Thr Pro Arg Thr Glu
            100                 105                 110
Glu Lys Tyr Lys Lys Ile Asn Glu Glu Phe Asp Asn Met Ile Lys Ser
        115                 120                 125
His Lys Ile Pro Ala Val Pro Pro Pro Asn Phe Glu Met Pro Val Thr
    130                 135                 140
Ile Pro Val Ser Ser His Asn Ser Leu Val Tyr Ser Asn Pro Val Ser
145                 150                 155                 160
Ser Leu Gly Asn Pro Asn Leu Leu Pro Leu Ala His Pro Ser Leu Gln
                165                 170                 175
Arg Asn Ser Met Ser Pro Gly Val Thr His Arg Pro Pro Ser Ala Gly
            180                 185                 190
Asn Thr Gly Gly Leu Met Gly Gly Asp Leu Thr Ser Gly Ala Gly Thr
        195                 200                 205

Ser Ala Gly Asn Gly Tyr Gly Asn Pro Arg Asn Ser Pro Gly Leu Leu
    210                 215                 220
Val Ser Pro Gly Asn Leu Asn Lys Asn Ile Gln Ala Lys Ser Pro Pro
225                 230                 235                 240
Pro Met Asn Leu Gly Met Asn Asn Arg Lys Pro Asp Leu Arg Val Leu
                245                 250                 255
Ile Pro Pro Gly Ser Lys Asn Thr Met Pro Ser Val Asn Gln Arg Ile
            260                 265                 270
Asn Asn Ser Gln Ser Ala Gln Ser Leu Ala Thr Pro Val Val Ser Val
        275                 280                 285
Ala Thr Pro Thr Leu Pro Gly Gln Gly Met Gly Gly Tyr Pro Ser Ala
    290                 295                 300
Ile Ser Thr Thr Tyr Gly Thr Glu Tyr Ser Leu Ser Ser Ala Asp Leu
305                 310                 315                 320
Ser Ser Leu Ser Gly Phe Asn Thr Ala Ser Ala Leu His Leu Gly Ser
                325                 330                 335
Val Thr Gly Trp Gln Gln Gln His Leu His Asn Met Pro Pro Ser Ala
            340                 345                 350
Leu Ser Gln Leu Gly Asp Arg Thr Thr Thr Pro Ser Arg Tyr Pro Gln
        355                 360                 365
His Thr Arg His Glu Ala Gly Arg Ser Pro Val Asp Ser Leu Ser Ser
    370                 375                 380
Cys Ser Ser Ser Tyr Asp Gly Ser Asp Arg Glu Asp His Arg Asn Glu
385                 390                 395                 400
Phe His Ser Pro Ile Gly Leu Thr Arg Pro Ser Pro Asp Glu Arg Glu
                405                 410                 415
Ser Pro Ser Val Lys Arg Met Arg Leu Ser Glu Gly Trp Ala Thr
            420                 425                 430

<210> 4
<211> 1395
<212> DNA
<213> Rat

<400> 4
atggggagaa aaaagattca gatcacgagg attatggatg aacgtaacag acaggtgact     60

tttacaaaga ggaaattcgg actgatgaag aaggcttatg agttgagcgt gctgtgcgac 120
tgtgagattg ccctgatcat cttcaacagc accaacaagc tgttccagta cgccagcacc 180
gacatggaca aggtgctgct caagtacacc gagtacaacg agccgcacga gagccggaca 240
aactcggaca ttgtggagac attgagaaag aagggcctta atggttgtga cagccccgat 300
cccgatgcag acgattcagt aggtcacagc cctgagtctg aggacaagta caggaaaatt 360
aacgaagata ttgatctaat gatcagcagg caaagattgt gtgctgttcc acctcccaac 420
tttgagatgc cagttaccat cccagtgtcc agtcataaca gtttggtata cagcaaccct 480
gtcagctcac tgggaaaccc taatcttctg ccactggccc acccttctct gcagaggaat 540
agtatgtctc ctggtgtgac acatagacct ccaagtgcag gtaacacagg tggtctgatg 600
ggtggagacc tcacatccgg tgcaggcacc agtgcaggga atggatacgg caacccccgg 660
aactcaccag gcctgctggt ctcacctggt aacctgaaca agaatataca agccaaatct 720
cctcccccta tgaatctagg aatgaataat cgtaagccag atctccgcgt cctcatcccc 780
cctggcagca agaacacaat gccatcagtg aatcaaagga taaataactc ccagtcggcc 840
cagtcattgg ctaccccagt ggtttctgta gcaactccta ctttaccagg acaaggaatg 900
ggaggatatc cgtcagccat ttcaacaaca tatggtaccg aatactctct gagcagcgca 960
gatctgtcat ctctgtctgg cttcaatact gccagtgcgc tccacctcgg ctccgtcact 1020
ggctggcagc agcagcacct acataacatg ccgccatctg ccctcagtca gttgggagct 1080
tgcactagca ctcatttatc tcagagttca aatctctccc tgccttctac tcaaagcctc 1140
aacatcaagt cagaacctgt ttctcctcct agagaccgta ccaccacccc atcgagatac 1200
ccacaacaca cgcgccacga ggcggggaga tctcctgttg acagcctgag cagctgtagc 1260
agttcctacg atgggagcga ccgggaggat caccggaacg aattccactc ccccattgga 1320
ctcaccagac cttcgccgga cgaaagggaa agtccctcag tcaagcgcat gcggctctct 1380
gaaggatggg caaca 1395

<210> 5
<211> 1299
<212> DNA
<213> Rat

<400> 5
atggggagaa aaaagattca gatcacgagg attatggatg aacgtaacag acaggtgact 60
tttacaaaga ggaaattcgg actgatgaag aaggcttatg agttgagcgt gctgtgcgac 120
tgtgagattg ccctgatcat cttcaacagc accaacaagc tgttccagta cgccagcacc 180
gacatggaca aggtgctgct caagtacacc gagtacaacg agccgcacga gagccggaca 240
aactcggaca ttgtggagac attgagaaag aagggcctta atggttgtga cagccccgat 300
cccgatgcag acgattcagt aggtcacagc cctgagtctg aggacaagta caggaaaatt 360

aacgaagata ttgatctaat gatcagcagg caaagattgt gtgctgttcc acctcccaac 420
tttgagatgc cagttaccat cccagtgtcc agtcataaca gtttggtata cagcaaccct 480
gtcagctcac tgggaaaccc taatcttctg ccactggccc acccttctct gcagaggaat 540
agtatgtctc ctggtgtgac acatagacct ccaagtgcag gtaacacagg tggtctgatg 600
ggtggagacc tcacatccgg tgcaggcacc agtgcaggga atggatacgg caacccccgg 660
aactcaccag gcctgctggt ctcacctggt aacctgaaca agaatataca agccaaatct 720
cctcccccta tgaatctagg aatgaataat cgtaagccag atctccgcgt cctcatcccc 780
cctggcagca agaacacaat gccatcagtg aatcaaagga taaataactc ccagtcggcc 840
cagtcattgg ctaccccagt ggtttctgta gcaactccta ctttaccagg acaaggaatg 900
ggaggatatc cgtcagccat ttcaacaaca tatggtaccg aatactctct gagcagcgca 960
gatctgtcat ctctgtctgg cttcaatact gccagtgcgc tccacctcgg ctccgtcact 1020
ggctggcagc agcagcacct acataacatg ccgccatctg ccctcagtca gttgggagac 1080
cgtaccacca ccccatcgag atacccacaa cacacgcgcc acgaggcggg gagatctcct 1140
gttgacagcc tgagcagctg tagcagttcc tacgatggga gcgaccggga ggatcaccgg 1200
aacgaattcc actcccccat tggactcacc agaccttcgc cggacgaaag ggaaagtccc 1260
tcagtcaagc gcatgcggct ctctgaagga tgggcaaca 1299

<210> 6
<211> 1293
<212> DNA
<213> Rat

<400> 6
atggggagaa aaaagattca gatcacgagg attatggatg aacgtaacag acaggtgact 60
tttacaaaga ggaaattcgg actgatgaag aaggcttatg agttgagcgt gctgtgcgac 120
tgtgagattg ccctgatcat cttcaacagc accaacaagc tgttccagta cgccagcacc 180
gacatggaca aggtgctgct caagtacacc gagtacaacg agccgcacga gagccggaca 240
aactcggaca ttgtggaggc attgaacaag aaagaaaaca aaggctctga aagccctgat 300
cctgactcct cttacgcact caccccacgc actgaagaaa aatacaaaaa aattaatgaa 360
gaatttgata atatgatcaa gagtcataaa attcctgctg ttccacctcc caactttgag 420
atgccagtta ccatcccagt gtccagtcat aacagtttgg tatacagcaa ccctgtcagc 480
tcactgggaa accctaatct tctgccactg gcccaccctt ctctgcagag gaatagtatg 540
tctcctggtg tgacacatag acctccaagt gcaggtaaca caggtggtct gatgggtgga 600
gacctcacat ccggtgcagg caccagtgca gggaatggat acggcaaccc ccggaactca 660
ccaggcctgc tggtctcacc tggtaacctg aacaagaata tacaagccaa atctcctccc 720
cctatgaatc taggaatgaa taatcgtaag ccagatctcc gcgtcctcat cccccctggc 780

agcaagaaca caatgccatc agtgaatcaa aggataaata actcccagtc ggcccagtca 840
ttggctaccc cagtggtttc tgtagcaact cctactttac caggacaagg aatgggagga 900
tatccgtcag ccatttcaac aacatatggt accgaatact ctctgagcag cgcagatctg 960
tcatctctgt ctggcttcaa tactgccagt gcgctccacc tcggctccgt cactggctgg 1020
cagcagcagc acctacataa catgccgcca tctgccctca gtcagttggg agaccgtacc 1080
accaccccat cgagataccc acaacacacg cgccacgagg cggggagatc tcctgttgac 1140
agcctgagca gctgtagcag ttcctacgat gggagcgacc gggaggatca ccggaacgaa 1200
ttccactccc ccattggact caccagacct tcgccggacg aaagggaaag tccctcagtc 1260
aagcgcatgc ggctctctga aggatgggca aca 1293

<210> 7
<211> 293
<212> DNA
<213> Rat

<400> 7
cggccgcaaa ggttttgaca gccccgatcc cgatgcagac gattcagtag gtcacagccc 60
tgagtctgag gacaagtaca ggaaaattaa cgaagatatt gatctaatga tcagcaggca 120
aagattgtgt gctgttccac ctcccaactt tgagatgcca gttaccatcc cagtgtccag 180
tcataacagt ttggtataca gcaaccctgt cagctcactg ggaaacccta atcttctgcc 240
actggcccac ccttctctgc agaggaatag tatgtctcct ggtgtgacac ata 293

<210> 8
<211> 27
<212> DNA
<213> Artificial Sequence

<220>
<223> Primer

<400> 8
ccatcctaat acgactcact atagggc 27

<210> 9
<211> 30
<212> DNA

<213> Artificial Sequence

<220>
<223> Primer

<400> 9
ctgcatcggg atcggggctg tcaaaacctt          30

<210> 10
<211> 22
<212> DNA
<213> Artificial Sequence

<220>
<223> Primer

<400> 10
gatcccgatg cagacgattc ag          22

<210> 11
<211> 24
<212> DNA
<213> Artificial Sequence

<220>
<223> Primer

<400> 11
tgcagctttg aatcaggtgg agaa          24

<210> 12
<211> 27
<212> DNA
<213> Artificial Sequence

<220>

<223> Primer

<400> 12
agtgggtatg gtcctctttg aatggtt          27

<210> 13
<211> 20
<212> DNA
<213> Artificial Sequence

<220>
<223> Primer

<400> 13
taatacgact cactataggg          20

<210> 14
<211> 18
<212> DNA
<213> Artificial Sequence

<220>
<223> Primer

<400> 14
atttaggtga cactatag          18

<210> 15
<211> 473
<212> PRT
<213> Rat

<400> 15
Met Gly Arg Lys Lys Ile Gln Ile Thr Arg Ile Met Asp Glu Arg Asn
                5                   10                  15
Arg Gln Val Thr Phe Thr Lys Arg Lys Phe Gly Leu Met Lys Lys Ala

            20                  25                  30
Tyr Glu Leu Ser Val Leu Cys Asp Cys Glu Ile Ala Leu Ile Ile Phe
        35                  40                  45
Asn Ser Thr Asn Lys Leu Phe Gln Tyr Ala Ser Thr Asp Met Asp Lys
    50                  55                  60
Val Leu Leu Lys Tyr Thr Glu Tyr Asn Glu Pro His Glu Ser Arg Thr
65                  70                  75                  80
Asn Ser Asp Ile Val Glu Thr Leu Arg Lys Lys Gly Leu Asn Gly Cys
                85                  90                  95
Asp Ser Pro Asp Pro Asp Ala Asp Asp Ser Val Gly His Ser Pro Glu
            100                 105                 110
Ser Glu Asp Lys Tyr Arg Lys Ile Asn Glu Asp Ile Asp Leu Met Ile
        115                 120                 125
Ser Arg Gln Arg Leu Cys Ala Val Pro Pro Pro Asn Phe Glu Met Pro
    130                 135                 140
Val Thr Ile Pro Val Ser Ser His Asn Ser Leu Val Tyr Ser Asn Pro
145                 150                 155                 160
Val Ser Ser Leu Gly Asn Pro Asn Leu Leu Pro Leu Ala His Pro Ser
                165                 170                 175
Leu Gln Arg Asn Ser Met Ser Pro Gly Val Thr His Arg Pro Pro Ser
            180                 185                 190
Ala Gly Asn Thr Gly Gly Leu Met Gly Gly Asp Leu Thr Ser Gly Ala
        195                 200                 205
Gly Thr Ser Ala Gly Asn Gly Tyr Gly Asn Pro Arg Asn Ser Pro Gly
    210                 215                 220
Leu Leu Val Ser Pro Gly Asn Leu Asn Lys Asn Ile Gln Ala Lys Ser
225                 230                 235                 240
Pro Pro Pro Met Asn Leu Gly Met Asn Asn Arg Lys Pro Asp Leu Arg
                245                 250                 255
Val Leu Ile Pro Pro Gly Ser Lys Asn Thr Met Pro Ser Val Ser Glu
            260                 265                 270
Asp Val Asp Leu Leu Leu Asn Gln Arg Ile Asn Asn Ser Gln Ser Ala
        275                 280                 285
Gln Ser Leu Ala Thr Pro Val Val Ser Val Ala Thr Pro Thr Leu Pro
    290                 295                 300
Gly Gln Gly Met Gly Gly Tyr Pro Ser Ala Ile Ser Thr Thr Tyr Gly

305                 310                 315                 320
Thr Glu Tyr Ser Leu Ser Ser Ala Asp Leu Ser Ser Leu Ser Gly Phe
                325                 330                 335
Asn Thr Ala Ser Ala Leu His Leu Gly Ser Val Thr Gly Trp Gln Gln
            340                 345                 350
Gln His Leu His Asn Met Pro Pro Ser Ala Leu Ser Gln Leu Gly Ala
        355                 360                 365
Cys Thr Ser Thr His Leu Ser Gln Ser Ser Asn Leu Ser Leu Pro Ser
    370                 375                 380
Thr Gln Ser Leu Asn Ile Lys Ser Glu Pro Val Ser Pro Pro Arg Asp
385                 390                 395                 400
Arg Thr Thr Thr Pro Ser Arg Tyr Pro Gln His Thr Arg His Glu Ala
                405                 410                 415
Gly Arg Ser Pro Val Asp Ser Leu Ser Ser Cys Ser Ser Ser Tyr Asp
            420                 425                 430
Gly Ser Asp Arg Glu Asp His Arg Asn Glu Phe His Ser Pro Ile Gly
        435                 440                 445
Leu Thr Arg Pro Ser Pro Asp Glu Arg Glu Ser Pro Ser Val Lys Arg
    450                 455                 460
Met Arg Leu Ser Glu Gly Trp Ala Thr
465                 470

<210> 16
<211> 441
<212> PRT
<213> Rat

<400> 16
Met Gly Arg Lys Lys Ile Gln Ile Thr Arg Ile Met Asp Glu Arg Asn
                5                   10                  15
Arg Gln Val Thr Phe Thr Lys Arg Lys Phe Gly Leu Met Lys Lys Ala
            20                  25                  30
Tyr Glu Leu Ser Val Leu Cys Asp Cys Glu Ile Ala Leu Ile Ile Phe
        35                  40                  45
Asn Ser Thr Asn Lys Leu Phe Gln Tyr Ala Ser Thr Asp Met Asp Lys
    50                  55                  60

Val Leu Leu Lys Tyr Thr Glu Tyr Asn Glu Pro His Glu Ser Arg Thr
65                  70                  75                  80
Asn Ser Asp Ile Val Glu Thr Leu Arg Lys Lys Gly Leu Asn Gly Cys
                85                  90                  95
Asp Ser Pro Asp Pro Asp Ala Asp Asp Ser Val Gly His Ser Pro Glu
            100                 105                 110
Ser Glu Asp Lys Tyr Arg Lys Ile Asn Glu Asp Ile Asp Leu Met Ile
        115                 120                 125
Ser Arg Gln Arg Leu Cys Ala Val Pro Pro Pro Asn Phe Glu Met Pro
    130                 135                 140
Val Thr Ile Pro Val Ser Ser His Asn Ser Leu Val Tyr Ser Asn Pro
145                 150                 155                 160
Val Ser Ser Leu Gly Asn Pro Asn Leu Leu Pro Leu Ala His Pro Ser
                165                 170                 175
Leu Gln Arg Asn Ser Met Ser Pro Gly Val Thr His Arg Pro Pro Ser
            180                 185                 190
Ala Gly Asn Thr Gly Gly Leu Met Gly Gly Asp Leu Thr Ser Gly Ala
        195                 200                 205
Gly Thr Ser Ala Gly Asn Gly Tyr Gly Asn Pro Arg Asn Ser Pro Gly
    210                 215                 220
Leu Leu Val Ser Pro Gly Asn Leu Asn Lys Asn Ile Gln Ala Lys Ser
225                 230                 235                 240
Pro Pro Pro Met Asn Leu Gly Met Asn Asn Arg Lys Pro Asp Leu Arg
                245                 250                 255
Val Leu Ile Pro Pro Gly Ser Lys Asn Thr Met Pro Ser Val Ser Glu
            260                 265                 270
Asp Val Asp Leu Leu Leu Asn Gln Arg Ile Asn Asn Ser Gln Ser Ala
        275                 280                 285
Gln Ser Leu Ala Thr Pro Val Val Ser Val Ala Thr Pro Thr Leu Pro
    290                 295                 300
Gly Gln Gly Met Gly Gly Tyr Pro Ser Ala Ile Ser Thr Thr Tyr Gly
305                 310                 315                 320
Thr Glu Tyr Ser Leu Ser Ser Ala Asp Leu Ser Ser Leu Ser Gly Phe
                325                 330                 335
Asn Thr Ala Ser Ala Leu His Leu Gly Ser Val Thr Gly Trp Gln Gln
            340                 345                 350

Gln His Leu His Asn Met Pro Pro Ser Ala Leu Ser Gln Leu Gly Asp
        355                 360                 365
Arg Thr Thr Thr Pro Ser Arg Tyr Pro Gln His Thr Arg His Glu Ala
    370                 375                 380
Gly Arg Ser Pro Val Asp Ser Leu Ser Ser Cys Ser Ser Ser Tyr Asp
385                 390                 395                 400
Gly Ser Asp Arg Glu Asp His Arg Asn Glu Phe His Ser Pro Ile Gly
                405                 410                 415
Leu Thr Arg Pro Ser Pro Asp Glu Arg Glu Ser Pro Ser Val Lys Arg
            420                 425                 430
Met Arg Leu Ser Glu Gly Trp Ala Thr
        435                 440

<210> 17
<211> 393
<212> PRT
<213> Rat

<400> 17
Met Gly Arg Lys Lys Ile Gln Ile Thr Arg Ile Met Asp Glu Arg Asn
                5                   10                  15
Arg Gln Val Thr Phe Thr Lys Arg Lys Phe Gly Leu Met Lys Lys Ala
            20                  25                  30
Tyr Glu Leu Ser Val Leu Cys Asp Cys Glu Ile Ala Leu Ile Ile Phe
        35                  40                  45
Asn Ser Thr Asn Lys Leu Phe Gln Tyr Ala Ser Thr Asp Met Asp Lys
    50                  55                  60
Val Leu Leu Lys Tyr Thr Glu Tyr Asn Glu Pro His Glu Ser Arg Thr
65                  70                  75                  80
Asn Ser Asp Ile Val Glu Ala Val Pro Pro Pro Asn Phe Glu Met Pro
                85                  90                  95
Val Thr Ile Pro Val Ser Ser His Asn Ser Leu Val Tyr Ser Asn Pro
            100                 105                 110
Val Ser Ser Leu Gly Asn Pro Asn Leu Leu Pro Leu Ala His Pro Ser
        115                 120                 125
Leu Gln Arg Asn Ser Met Ser Pro Gly Val Thr His Arg Pro Pro Ser

130                 135                 140
Ala Gly Asn Thr Gly Gly Leu Met Gly Gly Asp Leu Thr Ser Gly Ala
145                 150                 155                 160
Gly Thr Ser Ala Gly Asn Gly Tyr Gly Asn Pro Arg Asn Ser Pro Gly
                165                 170                 175
Leu Leu Val Ser Pro Gly Asn Leu Asn Lys Asn Ile Gln Ala Lys Ser
            180                 185                 190
Pro Pro Pro Met Asn Leu Gly Met Asn Asn Arg Lys Pro Asp Leu Arg
        195                 200                 205
Val Leu Ile Pro Pro Gly Ser Lys Asn Thr Met Pro Ser Val Ser Glu
    210                 215                 220
Asp Val Asp Leu Leu Leu Asn Gln Arg Ile Asn Asn Ser Gln Ser Ala
225                 230                 235                 240
Gln Ser Leu Ala Thr Pro Val Val Ser Val Ala Thr Pro Thr Leu Pro
                245                 250                 255
Gly Gln Gly Met Gly Gly Tyr Pro Ser Ala Ile Ser Thr Thr Tyr Gly
            260                 265                 270
Thr Glu Tyr Ser Leu Ser Ser Ala Asp Leu Ser Ser Leu Ser Gly Phe
        275                 280                 285
Asn Thr Ala Ser Ala Leu His Leu Gly Ser Val Thr Gly Trp Gln Gln
    290                 295                 300
Gln His Leu His Asn Met Pro Pro Ser Ala Leu Ser Gln Leu Gly Asp
305                 310                 315                 320
Arg Thr Thr Thr Pro Ser Arg Tyr Pro Gln His Thr Arg His Glu Ala
                325                 330                 335
Gly Arg Ser Pro Val Asp Ser Leu Ser Ser Cys Ser Ser Ser Tyr Asp
            340                 345                 350
Gly Ser Asp Arg Glu Asp His Arg Asn Glu Phe His Ser Pro Ile Gly
        355                 360                 365
Leu Thr Arg Pro Ser Pro Asp Glu Arg Glu Ser Pro Ser Val Lys Arg
    370                 375                 380
Met Arg Leu Ser Glu Gly Trp Ala Thr
385                 390

<210> 18
<211> 1419

<212> DNA
<213> Rat

<400> 18
atggggagaa aaaagattca gatcacgagg attatggatg aacgtaacag acaggtgact 60
tttacaaaga ggaaattcgg actgatgaag aaggcttatg agttgagcgt gctgtgcgac 120
tgtgagattg ccctgatcat cttcaacagc accaacaagc tgttccagta cgccagcacc 180
gacatggaca aggtgctgct caagtacacc gagtacaacg agccgcacga gagccggaca 240
aactcggaca ttgtggagac attgagaaag aagggcctta atggttgtga cagccccgat 300
cccgatgcag acgattcagt aggtcacagc cctgagtctg aggacaagta caggaaaatt 360
aacgaagata ttgatctaat gatcagcagg caaagattgt gtgctgttcc acctcccaac 420
tttgagatgc cagttaccat cccagtgtcc agtcataaca gtttggtata cagcaaccct 480
gtcagctcac tgggaaaccc taatcttctg ccactggccc acccttctct gcagaggaat 540
agtatgtctc ctggtgtgac acatagacct ccaagtgcag gtaacacagg tggtctgatg 600
ggtggagacc tcacatccgg tgcaggcacc agtgcaggga atggatacgg caacccccgg 660
aactcaccag gcctgctggt ctcacctggt aacctgaaca agaatataca agccaaatct 720
cctcccccta tgaatctagg aatgaataat cgtaagccag atctccgcgt cctcatcccc 780
cctggcagca agaacacaat gccatcagtg tctgaggatg tggacttgct gttgaatcaa 840
aggataaata actcccagtc ggcccagtca ttggctaccc cagtggtttc tgtagcaact 900
cctactttac caggacaagg aatgggagga tatccgtcag ccatttcaac aacatatggt 960
accgaatact ctctgagcag cgcagatctg tcatctctgt ctggcttcaa tactgccagt 1020
gcgctccacc tcggctccgt cactggctgg cagcagcagc acctacataa catgccgcca 1080
tctgccctca gtcagttggg agcttgcact agcactcatt tatctcagag ttcaaatctc 1140
tccctgcctt ctactcaaag cctcaacatc aagtcagaac ctgtttctcc tcctagagac 1200
cgtaccacca ccccatcgag atacccacaa cacacgcgcc acgaggcggg gagatctcct 1260
gttgacagcc tgagcagctg tagcagttcc tacgatggga gcgaccggga ggatcaccgg 1320
aacgaattcc actcccccat tggactcacc agaccttcgc cggacgaaag ggaaagtccc 1380
tcagtcaagc gcatgcggct ctctgaagga tgggcaaca 1419

<210> 19
<211> 1323
<212> DNA
<213> Rat

<400> 19
atggggagaa aaaagattca gatcacgagg attatggatg aacgtaacag acaggtgact 60

tttacaaaga ggaaattcgg actgatgaag aaggcttatg agttgagcgt gctgtgcgac 120
tgtgagattg ccctgatcat cttcaacagc accaacaagc tgttccagta cgccagcacc 180
gacatggaca aggtgctgct caagtacacc gagtacaacg agccgcacga gagccggaca 240
aactcggaca ttgtggagac attgagaaag aagggcctta atggttgtga cagccccgat 300
cccgatgcag acgattcagt aggtcacagc cctgagtctg aggacaagta caggaaaatt 360
aacgaagata ttgatctaat gatcagcagg caaagattgt gtgctgttcc acctcccaac 420
tttgagatgc cagttaccat cccagtgtcc agtcataaca gtttggtata cagcaaccct 480
gtcagctcac tgggaaaccc taatcttctg ccactggccc acccttctct gcagaggaat 540
agtatgtctc ctggtgtgac acatagacct ccaagtgcag gtaacacagg tggtctgatg 600
ggtggagacc tcacatccgg tgcaggcacc agtgcaggga atggatacgg caacccccgg 660
aactcaccag gcctgctggt ctcacctggt aacctgaaca agaatataca agccaaatct 720
cctcccccta tgaatctagg aatgaataat cgtaagccag atctccgcgt cctcatcccc 780
cctggcagca agaacacaat gccatcagtg tctgaggatg tggacttgct gttgaatcaa 840
aggataaata actcccagtc ggcccagtca ttggctaccc cagtggtttc tgtagcaact 900
cctactttac caggacaagg aatgggagga tatccgtcag ccatttcaac aacatatggt 960
accgaatact ctctgagcag cgcagatctg tcatctctgt ctggcttcaa tactgccagt 1020
gcgctccacc tcggctccgt cactggctgg cagcagcagc acctacataa catgccgcca 1080
tctgccctca gtcagttggg agaccgtacc accaccccat cgagataccc acaacacacg 1140
cgccacgagg cggggagatc tcctgttgac agcctgagca gctgtagcag ttcctacgat 1200
gggagcgacc gggaggatca ccggaacgaa ttccactccc ccattggact caccagacct 1260
tcgccggacg aaagggaaag tccctcagtc aagcgcatgc ggctctctga aggatgggca 1320
aca 1323

<210> 20
<211> 1179
<212> DNA
<213> Rat

<400> 20
atggggagaa aaaagattca gatcacgagg attatggatg aacgtaacag acaggtgact 60
tttacaaaga ggaaattcgg actgatgaag aaggcttatg agttgagcgt gctgtgcgac 120
tgtgagattg ccctgatcat cttcaacagc accaacaagc tgttccagta cgccagcacc 180
gacatggaca aggtgctgct caagtacacc gagtacaacg agccgcacga gagccggaca 240
aactcggaca ttgtggaggc tgttccacct cccaactttg agatgccagt taccatccca 300
gtgtccagtc ataacagttt ggtatacagc aaccctgtca gctcactggg aaaccctaat 360
cttctgccac tggcccaccc ttctctgcag aggaatagta tgtctcctgg tgtgacacat 420

agacctccaa gtgcaggtaa cacaggtggt ctgatgggtg gagacctcac atccggtgca 480
ggcaccagtg cagggaatgg atacggcaac ccccggaact caccaggcct gctggtctca 540
cctggtaacc tgaacaagaa tatacaagcc aaatctcctc cccctatgaa tctaggaatg 600
aataatcgta agccagatct ccgcgtcctc atcccccctg gcagcaagaa cacaatgcca 660
tcagtgtctg aggatgtgga cttgctgttg aatcaaagga taaataactc ccagtcggcc 720
cagtcattgg ctaccccagt ggtttctgta gcaactccta ctttaccagg acaaggaatg 780
ggaggatatc cgtcagccat ttcaacaaca tatggtaccg aatactctct gagcagcgca 840
gatctgtcat ctctgtctgg cttcaatact gccagtgcgc tccacctcgg ctccgtcact 900
ggctggcagc agcagcacct acataacatg ccgccatctg ccctcagtca gttgggagac 960
cgtaccacca ccccatcgag atacccacaa cacacgcgcc acgaggcggg gagatctcct 1020
gttgacagcc tgagcagctg tagcagttcc tacgatggga gcgaccggga ggatcaccgg 1080
aacgaattcc actcccccat tggactcacc agaccttcgc cggacgaaag ggaaagtccc 1140
tcagtcaagc gcatgcggct ctctgaagga tgggcaaca 1179

<210> 21
<211> 473
<212> PRT
<213> Human

<400> 21

<210> 22
<211> 466
<212> PRT
<213> Mouse

<400> 22
Met Gly Arg Lys Lys Ile Gln Ile Thr Arg Ile Met Asp Glu Arg Asn
                5                   10                  15
Arg Gln Val Thr Phe Thr Lys Arg Lys Phe Gly Leu Met Lys Lys Ala
            20                  25                  30
Tyr Glu Leu Ser Val Leu Cys Asp Cys Glu Ile Ala Leu Ile Ile Phe
        35                  40                  45
Asn Ser Thr Asn Lys Leu Phe Gln Tyr Ala Ser Thr Asp Met Asp Lys
    50                  55                  60
Val Leu Leu Lys Tyr Thr Glu Tyr Asn Glu Pro His Glu Ser Arg Thr
65                  70                  75                  80
Asn Ser Asp Ile Val Glu Thr Leu Arg Lys Lys Gly Leu Asn Gly Cys
                85                  90                  95
Asp Ser Pro Asp Pro Asp Ala Asp Asp Ser Val Gly His Ser Pro Glu
            100                 105                 110
Ser Glu Asp Lys Tyr Arg Lys Ile Asn Glu Asp Ile Asp Leu Met Ile
        115                 120                 125
Ser Arg Gln Arg Leu Cys Ala Val Pro Pro Pro Ser Phe Glu Met Pro
    130                 135                 140
Val Thr Ile Pro Val Ser Ser His Asn Ser Leu Val Tyr Ser Asn Pro
145                 150                 155                 160
Val Ser Thr Leu Gly Asn Pro Asn Leu Leu Pro Leu Ala His Pro Ser

                165                 170                 175
Leu Gln Arg Asn Ser Met Ser Pro Gly Val Thr His Arg Pro Pro Ser
            180                 185                 190
Ala Gly Asn Thr Gly Gly Leu Met Gly Gly Asp Leu Thr Ser Gly Ala
        195                 200                 205
Gly Thr Ser Ala Gly Asn Gly Tyr Gly Asn Pro Arg Asn Ser Pro Gly
    210                 215                 220
Leu Leu Val Ser Pro Gly Asn Leu Asn Lys Asn Ile Gln Ala Lys Ser
225                 230                 235                 240
Pro Pro Pro Met Asn Leu Gly Met Asn Asn Arg Lys Pro Asp Leu Arg
                245                 250                 255
Val Leu Ile Pro Pro Gly Ser Lys Asn Thr Met Pro Ser Val Asn Gln
            260                 265                 270
Arg Ile Asn Asn Ser Gln Ser Ala Gln Ser Leu Ala Thr Pro Val Val
        275                 280                 285
Ser Val Ala Thr Pro Thr Leu Pro Gly Gln Gly Met Gly Gly Tyr Pro
    290                 295                 300
Ser Ala Ile Ser Thr Thr Tyr Gly Thr Glu Tyr Ser Leu Ser Ser Ala
305                 310                 315                 320
Asp Leu Ser Ser Leu Ser Gly Phe Asn Thr Ala Ser Ala Leu His Leu
                325                 330                 335
Gly Ser Val Thr Gly Trp Gln Gln Gln His Leu His Asn Met Pro Pro
            340                 345                 350
Ser Ala Leu Ser Gln Leu Gly Ala Cys Thr Ser Thr His Leu Ser Gln
        355                 360                 365
Ser Ser Asn Leu Ser Leu Pro Ser Thr Gln Ser Leu Ser Ile Lys Ser
    370                 375                 380
Glu Pro Val Ser Pro Pro Arg Asp Arg Thr Thr Thr Pro Ser Arg Tyr
385                 390                 395                 400
Pro Gln His Thr Thr Arg His Glu Ala Gly Arg Ser Pro Val Asp Ser
                405                 410                 415
Leu Ser Ser Cys Ser Ser Ser Tyr Asp Gly Ser Asp Arg Glu Asp His
            420                 425                 430
Arg Asn Glu Phe His Ser Pro Ile Gly Leu Thr Arg Pro Ser Pro Asp
        435                 440                 445
Glu Arg Glu Ser Pro Ser Val Lys Arg Met Arg Leu Ser Glu Gly Trp

450                 455                 460
Ala Thr
465

<210> 23
<211> 432
<212> PRT
<213> Mouse

<400> 23
Met Gly Arg Lys Lys Ile Gln Ile Thr Arg Ile Met Asp Glu Arg Asn
                5                   10                  15
Arg Gln Val Thr Phe Thr Lys Arg Lys Phe Gly Leu Met Lys Lys Ala
            20                  25                  30
Tyr Glu Leu Ser Val Leu Cys Asp Cys Glu Ile Ala Leu Ile Ile Phe
        35                  40                  45
Asn Ser Thr Asn Lys Leu Phe Gln Tyr Ala Ser Thr Asp Met Asp Lys
    50                  55                  60
Val Leu Leu Lys Tyr Thr Glu Tyr Asn Glu Pro His Glu Ser Arg Thr
65                  70                  75                  80
Asn Ser Asp Ile Val Glu Ala Leu Asn Lys Lys Glu Asn Lys Gly Ser
                85                  90                  95
Glu Ser Pro Asp Pro Asp Ser Ser Tyr Ala Leu Thr Pro Arg Thr Glu
            100                 105                 110
Glu Lys Tyr Lys Lys Ile Asn Glu Glu Phe Asp Asn Met Ile Lys Ser
        115                 120                 125
His Lys Ile Pro Ala Val Pro Pro Pro Ser Phe Glu Met Pro Val Thr
    130                 135                 140
Ile Pro Val Ser Ser His Asn Ser Leu Val Tyr Ser Asn Pro Val Ser
145                 150                 155                 160
Thr Leu Gly Asn Pro Asn Leu Leu Pro Leu Ala His Pro Ser Leu Gln
                165                 170                 175
Arg Asn Ser Met Ser Pro Gly Val Thr His Arg Pro Pro Ser Ala Gly
            180                 185                 190
Asn Thr Gly Gly Leu Met Gly Gly Asp Leu Thr Ser Gly Ala Gly Thr
        195                 200                 205

Ser Ala Gly Asn Gly Tyr Gly Asn Pro Arg Asn Ser Pro Gly Leu Leu
    210                 215                 220
Val Ser Pro Gly Asn Leu Asn Lys Asn Ile Gln Ala Lys Ser Pro Pro
225                 230                 235                 240
Pro Met Asn Leu Gly Met Asn Asn Arg Lys Pro Asp Leu Arg Val Leu
                245                 250                 255
Ile Pro Pro Gly Ser Lys Asn Thr Met Pro Ser Val Asn Gln Arg Ile
            260                 265                 270
Asn Asn Ser Gln Ser Ala Gln Ser Leu Ala Thr Pro Val Val Ser Val
        275                 280                 285
Ala Thr Pro Thr Leu Pro Gly Gln Gly Met Gly Gly Tyr Pro Ser Ala
    290                 295                 300
Ile Ser Thr Thr Tyr Gly Thr Glu Tyr Ser Leu Ser Ser Ala Asp Leu
305                 310                 315                 320
Ser Ser Leu Ser Gly Phe Asn Thr Ala Ser Ala Leu His Leu Gly Ser
                325                 330                 335
Val Thr Gly Trp Gln Gln Gln His Leu His Asn Met Pro Pro Ser Ala
            340                 345                 350
Leu Ser Gln Leu Gly Asp Arg Thr Thr Thr Pro Ser Arg Tyr Pro Gln
        355                 360                 365
His Thr Thr Arg His Glu Ala Gly Arg Ser Pro Val Asp Ser Leu Ser
    370                 375                 380
Ser Cys Ser Ser Ser Tyr Asp Gly Ser Asp Arg Glu Asp His Arg Asn
385                 390                 395                 400
Glu Phe His Ser Pro Ile Gly Leu Thr Arg Pro Ser Pro Asp Glu Arg
                405                 410                 415
Glu Ser Pro Ser Val Lys Arg Met Arg Leu Ser Glu Gly Trp Ala Thr
            420                 425                 430